(19) Europäisches Patentamt
European Patent Office
Office européen des brevets

(11) Publication number: **0 100 041**
**B1**

(12) **EUROPEAN PATENT SPECIFICATION**

(45) Date of publication of patent specification: **22.10.86**

(21) Application number: **83106993.5**

(22) Date of filing: **16.07.83**

(51) Int. Cl.⁴: **C 07 D 417/14,**
C 07 D 403/04,
A 61 K 31/425, A 61 K 31/395
// C07D401/04, C07D417/04,
C07D413/04

(54) **1-Sulfo-2-azetidinone derivatives, their production and use.**

(30) Priority: **20.07.82 JP 127333/82**

(43) Date of publication of application:
**08.02.84 Bulletin 84/06**

(45) Publication of the grant of the patent:
**22.10.86 Bulletin 86/43**

(84) Designated Contracting States:
**BE CH DE FR GB IT LI NL SE**

(56) References cited:
**EP-A-0 053 815**
**EP-A-0 053 816**

(73) Proprietor: **Takeda Chemical Industries, Ltd.**
**27, Doshomachi 2-chome Higashi-ku**
**Osaka-shi Osaka, 541 (JP)**

(72) Inventor: **Numata, Mitsuo**
**25-13, Ankojicho 4-chome**
**Takatsuki Osaka 569 (JP)**
Inventor: **Miyake, Akio**
**30-22, Ominemotomachi 2-chome**
**Hirakata Osaka 573 (JP)**
Inventor: **Ochiai, Michihiko**
**23-13, Senriyamahigashi 1-chome**
**Suita Osaka 565 (JP)**

(74) Representative: **von Kreisler, Alek, Dipl.-Chem. et al**
**Deichmannhaus am Hauptbahnhof**
**D-5000 Köln 1 (DE)**

**EP 0 100 041 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel 1-sulfo-2-azetidinone derivatives having antibacterial and beta-lactamase inhibitory activities.

It has recently been found that certain bacterial species produce novel antibiotic substances having a 1-sulfo-2-azetidinone skeleton [Nature, *289*, 590 (1981) and *291*, 489 (1981)].

Furthermore, 1-sulfo-2-azetidinone derivatives having, at the 4-position, a substituent which is bonded to the azetidine ring on other atom than a carbon atom (e.g. an acylated amino group etc.) have been disclosed in European laid-open patent application No. 53,815.

However, no such 1-sulfo-2-azetidinone derivative having, at the 4-position, a cyclic amido substituent has ever been known.

The present inventors intensively searched for 1-sulfo-2-azetidinone derivatives having a further new structure and excellent antibacterial activity and as a result succeeded in synthesizing novel 1-sulfo-2-azetidinones having, at the 4-position, a five- or six-membered ring-forming primary or secondary amido group which may contain at least one of nitrogen, sulfur and oxygen atoms as a ring-constituting element and found that said compounds have excellent antibacterial and beta-lactamase inhibitory activities. Based on such findings, they have completed the present invention.

Thus, the present invention relates to a 1-sulfo-2-azetidinone derivative of the formula:

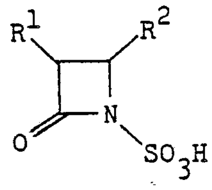

[I]

wherein $R^1$ is an amino group which may optionally be acylated with an acyl group commonly employed in β-lactam antibiotics or protected; $R^2$ is a five- or six-membered cyclic group represented by the formula:

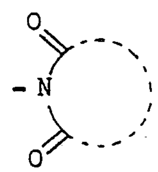

or

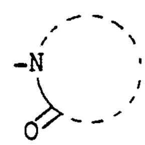

wherein the dotted arc portion may contain at least one of nitrogen, sulfur and oxygen as an arc-constituting member. The dotted arc portion being optionally condensed with a benzene ring, and the hydrogen atom or atoms on the dotted arc portion being optionally substituted by an alkyl group containing 1 to 4 carbon atoms, such as methyl, ethyl, propyl, isopropyl, butyl, isobutyl, sec-butyl and tert-butyl, a sulfo group or an oxo group.

More specifically, the amido group $R^2$ includes, among others, 2-oxopyrrolidin-1-yl, 2,5-dioxo-pyrrolidin-1-yl (succinimido), 5-oxo-2-pyrrolin-1-yl, 2,5-dioxo-3-pyrrolin-1-yl, 2-oxopiperidino, 2,5-dioxo-piperidino (glutarimido), 2-oxoindolin-1-yl, 3-oxoisoindolin-2-yl, 1,3-dioxoisoindolin-2-yl (phthalimido), 2-oxoimidazolidin-1-yl, 4,5-dioxoimidazolidin-1-yl, 2,5-dioxoimidazolidin-1-yl, 3,5-dioxopyrazolidin-1-yl, 2,3-dioxopiperazin-1-yl, 2,5-dioxopiperazin-1-yl, 4-oxothiazolidin-3-yl, 2,4-dioxothiazolidin-3-yl, 3-oxo-1,4-thiazan-4-yl, 2,4-dioxooxazolidin-3-yl and 2,5-dioxooxazolidin-3-yl. These may be substituted with a sulfo group or a lower alkyl group containing 1 to 4 carbon atoms as mentioned above.

Among these, preferred are, for example, 2,5-dioxopyrrolidin-1-yl, 1,3-dioxoisoindolin-2-yl, 2,5-dioxo-imidazolidin-1-yl and 2,3-dioxopiperazin-1-yl, each of which may be substituted with the above-mentioned lower alkyl group on its heterocyclic ring. Most preferred are, for instance, 2,5-dioxopyrrolidin-1-yl, 2,5-dioxoimidazolidin-1-yl, 2,5-dioxo-3-methylimidazolidin-1-yl, 1,3-dioxoisoindolin-2-yl and 2,3-dioxo-4-ethyl-piperazin-1-yl.

Referring to the above formula [1], the acyl moiety of the optionally acylated amino group represented by $R^1$ may be any of the acyl groups so far known as the acyl moieties in the 6-position side chain acylamino groups of penicillin derivatives or in the 7-position side chain acylamino groups of cephalosporin derivatives. In addition, the acyl groups disclosed in European laid open patent applications Nos. 53,815 and 53,816 and other acyl groups may also be used.

Among acyl groups such as mentioned above, those groups represented by the formula:

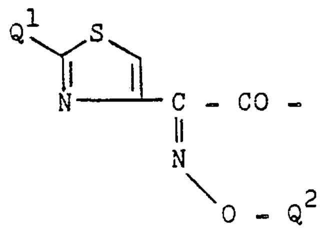

wherein $Q^1$ is an amino group which may be protected and $Q^2$ is a lower alkyl group, a lower alkenyl group, a group of the formula, $—CH_2COOQ^3$ or a group of the formula,

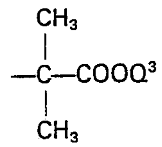

where $—COOQ^3$ is a carboxyl group which may optionally be esterified, are most preferably used.

Referring to groups represented by the above formulas, the amino-protecting group for the amino group which may optionally be protected ($Q^1$) is selected from among those amino-protecting groups that are mentioned hereinbelow in relation to $R^1$. The lower alkyl group represented by $Q^2$ is selected from among those lower alkyl groups that are mentioned hereinabove as the substituents on $R^2$. The lower alkenyl group represented by $Q^2$ includes lower alkenyl groups containing 2 to 4 carbon atoms, such as vinyl, allyl, 1-butenyl, 2-butenyl and 3-butenyl. The $COOQ^3$ group in the group of the formula, $—CH_2COOQ^3$ or

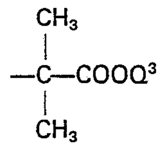

which is represented by $Q^2$, is a carboxyl group which may optionally be esterified, the ester residue represented by $Q^3$ being a carboxyl-protecting group which is used in the field of beta-lactam and peptide chemistry or a biologically active ester residue which can be easily removed *in vivo*. More specifically, said ester residue includes, among others, symmetric or unsymmetric tri-lower ($C_1$ to $C_4$) alkylsilyl groups, such as trimethylsilyl, methyldiethylsilyl and tri-tert-butylsilyl; lower($C_1$ to $C_4$) alkyl groups which may optionally be substituted with a halogen, a lower ($C_1$ to $C_4$) alkylsilyl or a lower ($C_1$ to $C_4$) alkylsulfonyl group, such as methyl, ethyl, propyl, isopropyl, n-butyl, isobutyl, sec-butyl, tert-butyl, β,β,β-trichloroethyl, β-trimethylsilylethyl, β-methylsulfonylethyl and β-ethylsulfonylethyl; aralkyl groups which may optionally be substituted with a nitro group, such as benzyl, phenethyl and p-nitrobenzyl; lower ($C_1$ to $C_4$) alkoxymethyl or lower ($C_1$ to $C_4$) alkoxyethyl groups, such as methoxymethyl, ethoxymethyl, propoxymethyl, isopropoxymethyl, α-methoxyethyl, α-ethoxyethyl, α-propoxyethyl and α-butoxyethyl; lower ($C_1$ to $C_4$) alkylthiomethyl groups, such as methylthiomethyl, ethylthiomethyl, isopropylthiomethyl and butylthiomethyl; lower ($C_2$ to $C_6$) alkanoyloxymethyl groups, such as acetoxymethyl and pivaloyloxymethyl; α-lower ($C_2$ to $C_6$) alkanoyloxy-α-lower ($C_1$ to $C_4$) alkyl-substituted methyl groups, such as α-acetoxypropyl, α-acetoxybutyl and α-pivaloyloxyethyl; lower ($C_1$ to $C_4$) alkoxycarbonylmethyl groups, such as methoxycarbonyloxymethyl and ethoxycarbonyloxymethyl; and lower ($C_1$ to $C_4$) alkoxycarbonyloxy-α-lower ($C_1$ to $C_4$) alkyl-substituted methyl groups, such as α-methoxycarbonyloxyethyl and α-ethoxycarbonyloxybutyl.

As the amino-protecting group for the amino group which may optionally be acylated or protected ($R^1$), there may be used any amino-protecting group which can be used in the field of beta-lactam and peptide chemistry and is easily removable and, in addition, there may be used adequately any of those easily-removable amino-protecting groups that are described by J. W. Barton in Chapter 2 of "Protective Groups in Organic Chemistry" edited by J. F. W. McOmie, Plenum Press, New York, 1973 or disclosed in European laid open patent application Nos. 53,815 and 53,816. Thus, the amino-protective group includes, among others, aliphatic acyl groups, such as formyl, monochloroacetyl and trifluoroacetyl; esterified carboxyl groups, such as tert-butoxycarbonyl, β,β,β-trichloroethoxycarbonyl, β-trimethylsilylethoxycarbonyl, β-methylsulfonylethoxycarbonyl, benzyloxycarbonyl, p-nitrobenzyloxycarbonyl, isobornyloxycarbonyl and phenyloxycarbonyl, and further phthaloyl, trityl, p-nitrobenzyl and proton etc., preferably trityl, phthaloyl and benzyloxy carbonyl.

Said protective groups are important for avoidance of undesired side-reactions in the production of the objective compound [I] and also in the step of production of the starting compound [III] of the present invention as mentioned hereinbelow.

Since the compound [I] has a sulfo group at the 1-position, it can generally react with a base to form a salt. Furthermore, when a carboxyl or sulfo group is presented in either or both of the substituents $R^1$ and $R^2$, such group can react with a base to form a salt. When an amino group is present, the amino group can react with an acid to form a salt. Therefore, the compound [I] may be obtained in the form of a pharmaceutically acceptable salt thereof. The salt obtained may be converted to a free form or to another pharmaceutically acceptable salt. Furthermore, the compound [I] obtained in the free form may be converted to a pharmaceutically acceptable salt thereof. The above-mentioned base includes, among others, inorganic bases, such as lithium, potassium, sodium, calcium and ammonia; basic amino acids, such as arginine, ornithine, lysine and histidine; and organic bases, such as N-methylglucamine, diethanolamine, triethanolamine and polyhydroxyalkylamines, e.g. tris(hydroxymethyl)aminomethane. The above-mentioned acid includes, among others, inorganic acids such as hydrochloric acid, sulfuric acid and phosphoric acid, and organic acids such as formic acid, acetic acid and p-toluenesulfonic acid.

The pharmaceutically acceptable salts of the compound [I] with the above-mentioned bases or acids also fall within the scope of the present invention. For converting the compound obtained in a salt form with a base to the free form, the method using an acid, for instance, may be used. The acid to be used includes inorganic acids, such as hydrochloric acid, sulfuric acid and phosphoric acid, and organic acids, such as formic acid, acetic acid and p-toluene-sulfonic acid, and such acids are frequently used. In addition, acid ion-exchange resins and the like may also be used. The solvents used in many cases are hydrophilic organic solvents, such as acetone, tetrahydrofuran, methanol, ethanol and dioxane; water; and a mixture thereof.

For the compound [I] or a pharmaceutically acceptable salt thereof which has substituents at 3- and 4-positions, there are *cis* and *trans* isomers. Furthermore, the carbon atoms at 3- and 4-positions are asymmetric carbon atoms. Accordingly, there can exist theoretically at least four stereoisomers (such as (3R,4R)-isomer, (3R,4S)-isomer, (3S,4R)-isomer, (3S,4S)-isomer. These isomers may be used either singly or in the form of a mixture. Among these isomers, (3S,4S)-isomer has most excellent antibacterial and beta-lactamase inhibitory activities. The same applies to the cases where the groups represented by $R^1$ and $R^2$ contain one or more asymmetric carbon atoms, and the resulting stereoisomers may be used either singly or in the form of a mixture.

The compound [I] or a pharmaceutically acceptable salt thereof is a valuable antibiotic active against various gram-positive and gram-negative bacteria and can be safely used as an antibacterial agent for the treatment of infections caused by gram-positive and gram-negative bacteria in man and mammals. As a bactericide, the compound [I] or a pharmaceutically acceptable salt is added to animal feed for preservation thereof. For inhibiting the troublesome bacterial growth on medical and dental devices and appliances and for inhibiting the troublesome bacterial growth in water-based or other paints, or in white water in paper mills or in other field of industry, it can be used as a bactericide in aqueous compositions in the concentration range of 0.1 to 100 parts by weight of antibiotic per million parts by weight of solution.

The compound [I] or a pharmaceutically acceptable salt thereof can be used in any of various pharmaceutical preparations either alone or in combination with other active ingredients. Thus, it can be incorporated in capsules, tablets, powders, solutions, suspensions and elixirs, for instance. These can be administered orally, intravenously or intramuscularly.

The tablets for oral administration may contain usual excipients, namely a binding agent such as syrup, gum arabic, gelatin, sorbitol, tragacanth gum or polyvinylpyrrolidone, a filler such as lactose, sugar, corn starch, calcium phosphate, sorbitol or glycine, a lubricant such as magnesium stearate, talc, polyethylene glycol or silica, a disintegration accelerator such as potato starch, carboxymethylcellulose calcium or sodium carboxystarch and a usable wetting agent such as sodium lauryl sulfate. The tablets can be coated by the methods well known in the art. The liquid preparations for oral aministration may take the form of aqueous or oily suspension, solution, emulsion, syrup, elixir or the like or the form of dry preparation to be dissolved in water or other adequate solvents prior to use. Such liquid preparations may contain a suspending agent such as sorbitol syrup, methylcellulose, glucose/sugar syrup, gelatin, hydroxyethylcellulose, carboxymethylcellulose, aluminum stearate gel or a hydrogenated edible oil such as almond oil, distillated coconut oil, a liquid ester, propylene glycol or ethyl alcohol, and a preservative such as methyl or propyl p-hydroxybenzoate, or sorbic acid. For preparing suppositories, conventional suppository bases such as cacao butter and other glycerides can be used.

The compositions for injection may be provided in unit dosage forms contained in ampules or in containers with a preservative added. Said compositions may be in the form of suspension, solution or emulsion in an oily or aqueous solvent and may contain adequate auxiliaries such as a suspending agent, a stabilizer and (or) a dispersing agent. The active ingredient may also be made into a powder form which is to be reconstructed with an adequate solvent, for instance sterilized, pyrogen-free water, prior to use.

It can also be made into an adequate form allowing its absorption through the nasal and guttural mucosa or the bronchial tissue, for instance, powder, liquid spray or inhalant, lozenge or throat paint. For administration to the eye or ear, it can be used in the form of liquid or semisolid in capsules or of drops. Furthermore, hydrophobic or hydrophilic bases may be used for making external preparations, such as ointment, cream, lotion, paint and powder.

The pharmaceutical preparations may also contain other ingredients than vehicles, for example a stabilizer, a binding agent, an antioxidant, a preservative, a lubricant, a suspending agent, a thickening

agent and/or a flavoring agent. Furthermore, the compositions may containe one or more of other active ingredients so as to provide a broader antimicrobial spectrum.

For use in domestic animals, preparation for administration into the mammary gland which comprise a base capable of prolonging the action of the active ingredient or of releasing the active ingredient rapidly can be formulated.

The compound [I] or a pharmaceutically acceptable salt thereof of the present invention can be used as a bacterial infection-treating agent in the treatment of respiratory tract infections, urinary tract infections, suppurative diseases, bile tract infections, enteral infections, infectious diseases in obstetrics and gynecology and surgical infections, among others. The daily dose depends on the conditions of the patient to be treated, the body weight of the host, the route and frequency of administration, parenteral administration being preferred for general infections and oral administration for enteral infections. Generally, the daily oral dose is about 20 to 600 mg of active ingredient per kg of body weight of the patient in single or multiple daily dosing. A suitable daily dose for adult human is within the range of about 20 to about 400 mg of active ingredient per kg of body weight and it is suitably administered parenterally in two to four divided doses daily, the single dose being about 5 to about 100 mg/kg.

The compositions containing the compound [I] or a pharmaceutically acceptable salt thereof can be administered in several unit dosage forms which are solid or liquid, for instance, and are administrable orally. The liquid or solid compositions in unit dosage forms contain 0.5 to 99% of the active substance. The preferred range is about 10 to 60%. Each unit dosage form generally contains about 15 to 1500 mg of the active ingredient. It is preferable, to select the amount within the range of about 250 to 1000 mg.

In addition to the above-mentioned mode of use, the compound [I] or a pharmaceutically acceptable salt thereof may be used in combination with a beta-lactam antibiotic since it has beta-lactamase inhibitory activity. Examples of such beta-lactam antibiotic are penicillins, such as benzylpenicillin, phenoxymethylpenicillin, carbenicillin, ampicillin, amoxicillin and sulbenicillin, and cephalosporins, such as cephaloridine, cephalothin, cefazolin, cephalexin, cefoxitin, cefacetrile, cefamandol, cefmenoxime, cefsulodine, cefotiam, cefotaxime, cefapirin, ceftizoxime, cephradine and cephaloglycine.

The 1-sulfo-2-oxoazetidine derivative [I], or a pharmaceutically acceptable salt thereof can be produced, for example, by sulfonating a compound of the formula:

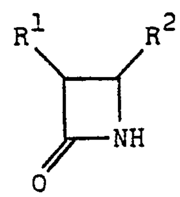

[II]

wherein the symbols are as defined above, or a salt or silyl derivative thereof.

The compound [II] can be used in the form of salts with various acids or bases, or silyl derivatives, among others. As the compound [II] contains substituents at 3- and 4-positions *cis-trans* isomerism occurs. Furthermore, the carbon atoms at 3- and 4-positions are asymmetric carbon atoms. Accordingly, there can exist theoretically at least four stereoisomers (such as (3R,4R)-isomer, (3R,4S)-isomer, (3S,4R)-isomer, (3S,4S)-isomer). These stereoisomers may be used either singly or in the form of a mixture. The same applies to the cases where an asymmetric carbon atom is contained in either or both of the groups represented by $R^1$ and $R^2$; the resulting stereoisomers may be used either singly or in the form of a mixture.

As the salts of the compound [II], use can be made of the salts as mentioned above for the compound [I], among others. The compound [II] may be silylated with a silylating agent. The silylating agent is, for example, a compound of the formula:

$$P^1P^2P^3Si{\cdot}Hal$$

wherein $P^1$, $P^2$ and $P^3$ each is a hydrocarbon group such as a lower alkyl group containing 1 to 4 carbon atoms (e.g. methyl, ethyl, n-propyl, isopropyl, n-butyl) or an aryl group (e.g. phenyl, tolyl), Hal is a halogen atom, preferably a chlorine or bromine atom, one or two of $P^1$, $P^2$ and $P^3$ may be each a halogen atom, preferably a chlorine or bromine atom, and furthermore one of $P^1$, $P^2$ and $P^3$ may be a hydrogen atom. Furthermore, hexa$(C_{1-4})$ alkylcyclotrisilazanes, octa$(C_{1-4})$ alkylcyclotetrasilazanes, tri$(C_{1-4})$ alkylsilylacetamides and bis-tri$(C_{1-4})$alkylsilylacetamides, for instance, may also be used as silylating agents. Preferred silylating agents are, for example, silyl compounds of the formula:

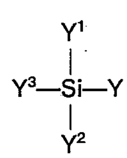

wherein $Y^1$ and $Y^2$ each are a lower alkyl, phenyl, benzyl or lower alkoxy group, $Y^3$ is a tert-butyl or isopropyl group and Y is a reactive group capable of leaving the silylating agent. In the above formula, the lower alkyl group represented by each of $Y^1$ and $Y^2$ contains 1 to 4 carbon atoms and is, for example, methyl, ethyl, n-propyl, isopropyl, n-butyl and tert-butyl, and the lower alkoxy group contains 1 to 4 carbon atoms and is, for example, methoxy, ethoxy, n-propoxy, isopropoxy, n-butoxy and tert-butoxy. The reactive group Y to be freed from the silylating agent is, for example, a halogen atom (e.g. chlorine, bromine) or further an N-(trialkylsilyl)trifluoroacetimidoyloxy group; an N-(trialkylsilyl)acetimidoyloxy group; an acylamino group such as formylamino, acetylamino, propionylamino, butyrylamino and trifluoroacetylamino; a (trialkylsilyl)amino group such as (tri-tert-butyldimethylsilyl)amino, isopropyldimethylsilylamino and (chloromethyldimethylsilyl)amino; amino; an alkylamino group such as methylamino, ethylamino and propylamino; a N,N-dialkylamino group such as N,N-dimethylamino, N-chloromethyl-N-methylamino, N,N-diethylamino, N,N-dipropylamino, N-methyl-N-ethylamino, N-methyl-N-propylamino and N-ethyl-N-propylamino; and a heterocyclic group such as imidazolyl. The alkyl group in such reactive group Y is a lower ($C_1$ to $C_4$) alkyl group such as methyl, ethyl, n-propyl, isopropyl, n-butyl and tert-butyl.

Specific examples of such silyl compound are N,O-bis(tert-butyldimethylsilyl)trifluoroacetamide, N,O-bis(isopropyldimethylsilyl)acetamide, bis(dimethylisopropylsilyl)acetamide, isopropyldimethylsilylacetamide, bis(dimethyl-tert-butylsilyl)acetamide, N-methyl-N-tert-butyldimethylsilylacetamide, N-methyl-N-isopropyldimethylsilyltrifluoroacetamide, N-tert-butyldimethylsilyldiethylamine, 1,3-bis(chloromethyl)-1,1,3,3-tetra-tert-butyldimethyldisilazane, N-isopropyldimethylsilylimidazole, tert-butyldiphenylchlorosilane, isopropyldiethylchlorosilane, tert-butyldimethylchlorosilane, isopropyldimethylchlorosilane and tert-butyldiethylchlorosilane.

When, among these, tert-butyldimethylchlorosilane, isopropyldimethylchlorosilane, and the like are used, the silyl derivatives can be isolated in stable state. The silylation reaction is carried out in the temperature range of about 0°C to 50°C, preferably about 0°C to 38°C, normally at room temperature (about 20°C). The reaction time is several minutes (about 10 minutes) to 24 hours. The reaction is conveniently conducted in a solvent inert to the reaction, such as ethyl acetate, dioxane, tetrahydrofuran, N,N-dimethylacetamide, N,N-dimethylformamide, dichloromethane, chloroform, benzene, toluene, acetone, methyl ethyl ketone and acetonitrile, or a mixture thereof. The reaction may be carried out in the presence of an inorganic base such as sodium hydroxide, potassium hydroxide, sodium hydrogen carbonate, sodium carbonate and potassium carbonate, or an organic base, such as trialkylamine (e.g. triethylamine, tributylamine), a triaralkylamine (e.g tribenzylamine), an organic tertiary amine (e.g. N-methylmorpholine, N-methylpiperidine, N,N-dialkylaniline, N,N-dialkylbenzylamine, pyridine, picoline, lutidine), 1,5-diazabicyclo[4,3,0]non-5-ene, 1,4-diazabicyclo[2,2,2]octane and 1,8-diazabicyclo[5,4,4]undecene-7. When the base is liquid, it may also serve as a solvent. The thus-obtained silyl derivative of the compound [II] is used as the starting material for the sulfonation reaction either as contained in the reaction mixture or after isolation and purification by the known method such as mentioned hereinbelow.

When, in the thus-obtained 1-silyl derivative of the compound [II], the above-mentioned $R^1$ is a protected amino group, it is also possible to produce the 1-silyl-3-acylamino derivative of the compound [II] by elimination of the protective group, followed by acylation. The above elimination of the protective group and acylation may be carried out by a *per se* known method as mentioned below. This exchange reaction of the substituents at 3-position can be carried out easily since the reaction proceeds smoothly to give good yield and requires only a simple procedure. Therefore, said reaction is very useful for the synthesis of various 2-oxoazetidine derivatives substituted by a desired acyl group. Furthermore, said reaction can be directly followed by the sulfonation reaction according to the present invention. Said sulfonation means the reaction for introducing a sulfo group into the 1-position of the compound [II].

The sulfonation reaction is carried out by reacting the compound [II], or a salt or silyl derivative thereof with sulfuric anhydride or a reactive derivative thereof as the sulfonating agent in a solvent. The reactive derivative of sulfuric anhydride includes, among others, sulfuric anhydride-base complexes, such as sulfuric anhydride-pyridine, sulfuric anhydride-picoline, sulfuric anhydride-lutidine and sulfuric anhydride-trimethylamine, and sulfuric anhydride-complexes such as sulfuric anhydride-dioxane, sulfuric anhydride-N,N-dimethylformamide and sulfuric anhydride-chlorosulfonic acid.

In carrying out the sulfonation reaction, sulfuric anhydride or a reactive derivative thereof is used in an amount of about 1 to 10 moles, preferably about 1 to 5 moles, per mole of the compound [II]. The reaction temperature is within the range of about −78°C to about 80°C, preferably about −20°C to about 60°C. Usable solvents are water and ordinary organic solvents such as ethers (e.g. dioxane, tetrahydrofuran, diethyl ether), esters (e.g. ethyl acetate, ethyl formate), halogenated hydrocarbons (e.g. chloroform, dichloromethane), hydrocarbons (e.g. benzene, toluene, n-hexane) and amides (e.g. N,N-dimethylformamide, N,N-dimethylacetamide), and these may be used either singly or in a mixture thereof. Depending on the starting compound [II], sulfonating agent, reaction temperature and solvent, the reaction is complete generally in several tens of minutes to several tens of hours, although several tens of days is required in some cases. After the reaction, the reaction mixture is subjected to a *per se* known separation/purification step, such as concentration, pH adjustment, solvent extraction, crystallization, recrystallization, fractional distillation and/or chromatography, whereby the compound [I] or a pharmaceutically acceptable salt thereof is isolated and purified to a desired purity.

The stereoisomers of the compound [II] may be subjected to the sulfonation reaction either singly or in the form of a mixture. When the product is a mixture of stereoisomers, each stereoisomer can be isolated as desired by column chromatography, recrystallization or some other technique.

The 1-sulfo-2-oxoazetidine derivative [I] wherein $R^{1}$ is an acylated amino group, that is a compound represented by the formula:

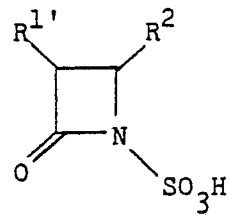

[I']

wherein $R^{1'}$ is an acylated amino group and $R^2$ is as defined above can be produced by acylating an 3-amino-2-azetidinone derivative of the formula:

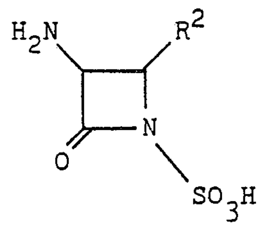

[III]

wherein $R^2$ is as defined above, or a salt or silyl derivative thereof. Referring to the formula [I'], an acylated amino group represented by $R^{1'}$ is as defined in $R^1$.

The acylation reaction is effected by reacting an acylating agent with the compound [III], or a salt or silyl derivative thereof, said acylating agent being used in an amount of not less than one mole, preferably about 1.2 to 4 moles, per mole of the compound [III]. The acylating agent to be used for the acylation reaction may be an organic carboxylic acid containing the acyl group represented by $R^{1'}$ [$R^oCOOH$ wherein $R^oCO$ is the same acyl group as contained in the acylated amino group represented by $R^{1'}$] or a reactive derivative thereof modified at the carboxyl group.

The salts and silyl derivatives of the compound [III] are respectively of the same kinds as those mentioned above for the compound [II].

The above-mentioned reactive derivative of organic acid includes, among others, acid halides, acid anhydrides, active amides, active esters and active thioesters. More specific examples of such reactive organic acid derivatives are as mentioned below.

1) Acid halides

As the acid halides, there are used acid chlorides and acid bromides, for instance.

2) Acid anhdyrides

As the acid anhydrides, there are used monoalkyl carbonic acid mixture anhydrides, mixed anhdyrides with aliphatic carboxylic acids (e.g. acetic acid, pivalic acid, valeric acid, isovaleric acid, trichloroacetic acid), mixed anhydrides with aromatic carboxylic acids (e.g. benzoic acid) and symmetric anhydrides, among others.

3) Active amides

As the active amides, there are used, for example, amides with pyrazole, imidazole, 4-substituted imidazole, dimethylpyrazole and benzotriazole.

4) Active esters

As the active esters, there are used, for instance, esters with 1-hydroxy-1H-2-pyridone, N-hydroxysuccinimide and N-hydroxyphthalimide as well as methyl esters, ethyl esters, methoxymethyl esters, propargyl esters, 4-nitrophenyl esters, 2,4-dinitrophenyl esters, trichlorophenyl esters, pentachlorophenyl esters and mesylphenyl esters.

5) Active thioesters

As the active thioester, there are used, for instance, thioesters with heterocyclic thiols such as 2-pyridinethiol and 2-benzothiazolethiol.

The reactive organic acid derivative to be used in each specific case should desirably be selected from among those such as mentioned above depending on the acid species employed. When a free acid is used as the acylating agent, the reaction is preferably carried out in the presence of a condensing agent. The condensing agent to be used in such case is, for example, N,N'-dicyclohexylcarbodiimide, N-cyclohexyl-N'-morpholinoethylcarbodiimide, N-cyclohexyl-N'-(4-diethylaminocyclohexyl)carbodiimide and N-ethyl-N'-(3-dimethylaminopropyl)carbodiimide.

Said acylation reaction is generally carried out in a solvent. Usable are water, acetone, dioxane, acetonitrile, methylene chloride, chloroform, dichloroethane, tetrahydrofuran, ethyl acetate, N,N-dimethyl

formamide, N,N-dimethylacetamide, pyridin and other conventional organic solvents which are inert to the reaction among these, those that are hydrophilic can also be used in a mixture with water.

The acylation reaction can also be conducted in the presence of a base such as an alkali metal carbonate, a trialkylamine (e.g. trimethylamine, triethylamine, tributylamine, N-methymorpholine, N-methylpiperidine), a N,N-dialkylaniline, a N,N-dialkylbenzylamine, pyridine, picoline, lutidine 1,5-diazabicyclo[4,3,0]non-5-ene, 1,4-diazabicyclo[2,2,2]octane and 1,8-diazabicyclo[5,4,4]undecene-7. The base or the above-mentioned condensing agent, when it is liquid, may also serve as the solvent. The reaction temperature is not critical. In many cases, the reaction is generally carried out under cooling or at room temperature. The reaction is complete in serveral minutes to several tens of hours. The thus-produced compound [I'] can be isolated and purified by *per se* known procedures, such as concentration, pH adjustment, solvent extraction, crystallization, recrystallization, fractional distillation and/or chromatography.

In cases where the starting compound [III] or a salt or silyl derivative thereof and/or the acylating agent contains an asymmetric carbon aotm in the molecule thereof, either each single stereoisomer or a mixture of stereoisomers may be subjected to the above acylation reaction. Furthermore, when the product is a mixture of stereoisomers, each individual stereoisomer can be isolated by the conventional method, for instance by column chromatography or recrystallization, as necessary.

In cases where the products [I], [I'] thus obtained by sulfonation or acylation have a protective group, the product can be subjected to deprotection reaction, followed by purification as necessary, whereby the desired compounds [I], [I'] are obtained. The deprotection method is selected from among *per se* known methods such as the method involving the use of an acid, one using a base, a reductive method, the method involving the use of hydrazine, and the method involving the use of thiourea or sodium N-methyldithiocarbamate. The method involving the use of an acid employs, such as an inorganic acid (e.g. hydrochloric acid, sulfuric acid, phosphoric acid etc.), an organic acid (e.g. formic acid, acetic acid, trifluoroacetic acid, propionic acid, benzenesulfonic acid, p-toluenesulfonic acid, etc.) and acid ion exchange resins and so on. The method involving the use of a base employs, such as an inorganic base such as the hydroxides or carbonates or alkali metal (e.g. sodium, potassium, etc.) or of alkaline earth metals (e.g. calcium magnesium, etc.), an organic base such as metal alkoxides, organic amines, quaternary ammonium salts, and basic ion exchange resins, etc.

When the above method involving the use of an acid or a base is carried out in the presence of a solvent, the solvent is usually a hydrophilic organic solvent, water or a mixed solvent.

The reductive method employs, according to the type of protective group and other conditions, a metal (e.g. tin, zinc, etc.) or a metal compound (e.g. chromous chloride, chromous acetate, etc.) together with an acid such as an organic or inorganic acid (e.g. acetic acid, propionic acid, hydrochloric acid), or involves the use of a metal catalyst for catalytic reduction. The catalyst used for such catalytic reduction may for example be platinum catalysts (e.g. platinum wire, platinum sponge, platinum black, platinum oxide, colloidal platinum, etc.), palladium catalysts (e.g. palladium sponge, palladium black, palladium oxide, palladium-barium sulfate, palladium-barium carbonate, palladium-carbon, palladium-silica gel, colloidal palladium, etc.), reduced nickel, nickel oxide, Raney nickel, and Urushihara nickel, etc.

The reductive method involving the use of a metal and an acid employs a metal compound (e.g. of iron or chromium) and an inorganic acid (e.g. hydrochloric acid) or organic acid (e.g. formic acid, acetic acid, propionic acid, etc.). The reductive method is usually conducted in a solvent. In the catalytic reduction method, for instance, the reaction is conducted usually in the presence of an alcohol (e.g. methanol, ethanol, propyl alcohol, isopropyl alcohol, etc.), ethyl acetate, etc. The method involving the use of a metal and an acid is usually carried out in the presence of water, acetone or the like, but when the acid is liquid, it may be utilized as the solvent as well.

The reaction is usually conducted in the range of from cooling to warming.

The compound which is protective group-free is isolated, in the free form or if necessary after conversion to a salt form, by column chromatography and/or recrystallization, for instance.

The starting compound [II] and [III] which are used in the present invention are novel compounds and can be produced, for instance, according to the method disclosed in European laid open patent appln. No. 53,815 or a modification thereof, or from a penicillin via the route illustrated below.

1)

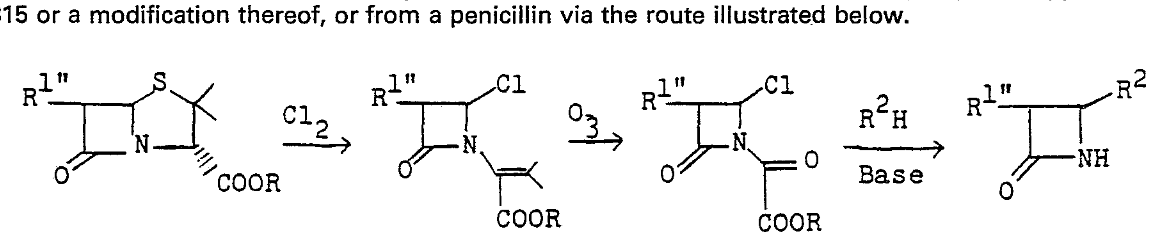


Penicillin ester [IV] [V] [VI]

In the formulas, $R^{1''}$ is a protected amino group such as phthalimido or benzyloxycarboxamido, $R^2$ is as

defined above and R is an ester residue such as a lower ($C_1$ to $C_4$) alkyl group (e.g. methyl, ethyl, propyl, isobutyl, n-butyl etc.)

2)

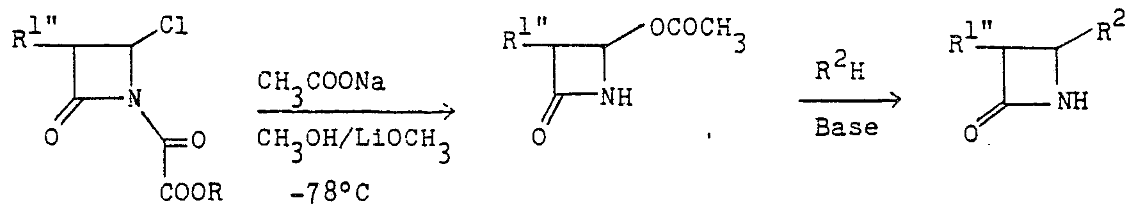


[VI] [VII]

In the formulas, the symbols are as defined above.

3)

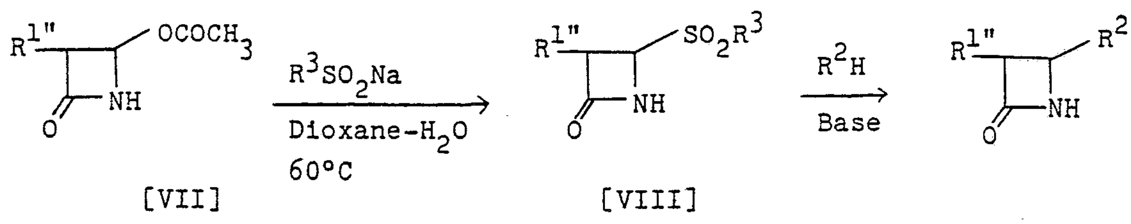


[VII] [VIII]

In the formulas, $R^{1''}$ and $R^2$ are as defined above and $R^3$ is, for instance, a lower ($C_1$ to $C_4$) alkyl group as defined in above 1) or an aryl group such as phenyl and phenethyl.

4)

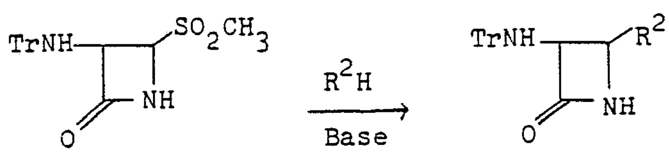


[VIII']

In the formulas, Tr is a trityl group and $R^2$ is as defined above.

5)

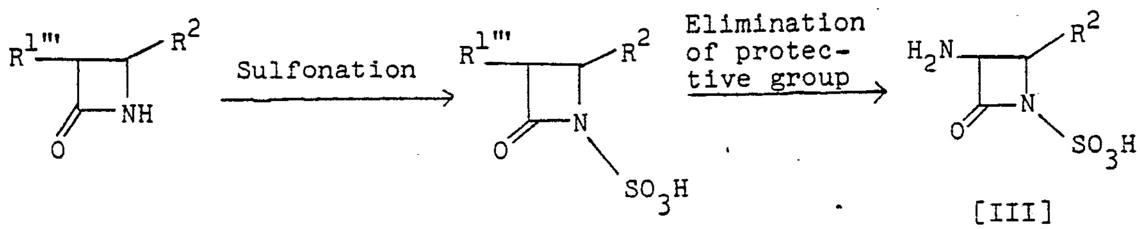


[III]

In the formulas, $R^{1'''}$ is, for example, a phthalimido, benzyloxycarboxamido or tritylamino group and $R^2$ is as defined above.

The method of producing the penicillin ester [IV], which is the starting material for the above-illustrated processes, is well known in the art. The method of producing the compound [VI] is described in European laid open patent appln. No. 52,299 and the method of producing the compound [VII] is described in Tetrahedron Letters, *1978*, 4059. The method of producing the starting materials [VIII], [VIII'] are described by E. G. Brain et al. in J. C. S. Perkin I, *1976*, 447.

The following Examples will illustrate the invention in more detail. In the Examples, the symbols used are defined as follows;

s, singlet; d, doublet; dd or d, d, double doublet; t, triplet; q, quartet; br or br., broad, DMSO, dimethyl sulfoxide.

In silicagel column-chromatography, Kiesel Gel 60 (Art 9385, 230—400 mesh, E. Merck AG., W. Germany) is employed and in a column-chromatography using Diaion CHP20P, Diaion CHP20P (produced by Mitsubishi Chemical Industries Ltd., Japan) is employed. In these column-chromatography, the elution in the chromatography is monitored by UVICORD (produced by LKB., Sweden).

Example 1

Production of sodium (3S,4S)-cis-3-[2-(2-aminothiazol-4-yl)-(Z)-2-methoxyimino]-acetamido-4-succinimido-2-azetidinone-1-sulfonate

(1) A mixture of 1.6 g of succinimide and 14 ml of 1M sodium methoxide-methanol is added dropwise to a dichloromethane solution (50 ml) containing 14.4 mMol of methyl 2-[(3R,4R)-trans-3-benzyloxycarboxamido-4-chloro-2-oxoazetidin-1-yl]-2-oxoacetate [described in Example 32, European laid open patent application No. 52,299] with stirring at −78°C. The mixture is stirred for about 2 minutes and then 1.5 ml of acetic acid is added. The reaction mixture is concentrated to dryness. The oily residue is then subjected to silica gel column chromatography, elution being carried out with ethyl acetate-n-hexane (2:1) to give 2.14 g of purified (3S,4S)-cis-3-benzyloxycarboxamido-4-succinimido-2-azetidinone.

mp 72 to 75°C

$[\alpha]_D^{24}$ + 10.8° (c = 1.13, $C_2H_5OH$)

IR spectrum $\nu_{max}^{KBr}$ $cm^{-1}$: 3320, 1780, 1700.

NMR spectrum ($CDCl_3$, 100MHz) δ: 2.64 (4H, s, $COCH_2CH_2CO$), 5.06 (2H, s, $OCH_2$), 5.44 (1H, dd, J=4.5 & 10Hz, 3-H), 5.77 (1H, d, J=10Hz, CONH), 5.78 (1H, d, J=4.5Hz, 4-H), 6.72 (1H, br, NH), 2.33 (5H, br, $C_6H_5$).

(2) 10% Sulfuric anhydride-dimethylformamide solution (10 ml) is added dropwise to a solution of 700 mg of (3S,4S)-cis-3-benzyloxycarboxamido-4-succinimido-2-azetidinone in 1.5 ml of dimethylformamide with stirring, under cooling at −20°C. The mixture is stirred for 3 hours, followed by dropwise addition of 5 ml of 10% sulfuric anhydride-dimethylformamide. The resulting mixture is stirred at 0°C overnight. Then, with stirring at −20°C, to the mixture is added 4 g of sodium hydrogen carbonate and the mixture is stirred at 0°C for 20 minutes. To the mixture is added ethyl ether and the resulting powdery precipitate is collected by filtration, washed with ether and air-dried. This powder is dissolved in 50 ml of water and the solution is subjected to a column-chromatography using Diaion CHP20P. The elution is then carried out with water, 5% ethanol and 10% ethanol in that order. The fractions containing the desired product are collected and lyophilized to give 410 mg of sodium (3S,4S)-cis-3-benzyloxycarboxamido-4-succinimido-2-azetidinone-1-sulfonate.

IR spectrum $\nu_{max}^{KBr}$ $cm^{-1}$: 3410, 1880, 1250, 1055.

(3) A mixture of 395 mg of sodium (3S,4S)-cis-3-benzyloxycarboxamido-4-succinimido-2-azetidinone-1-sulfonate, 400 mg of 10% palladium-on-carbon and 6 ml of water is stirred under a hydrogen atmosphere. After completion of the reaction (which requires about an hour), the mixture is filtered by suction. A mixture of this filtrate, 407 mg of sodium hydrogen carbonate and 10 ml of acetone is stirred under ice-cooling. To the mixture is added dropwise 444 mg of 2-(2-chloro-acetamidothiazol-4-yl)-(Z)-2-methoxyiminoacetyl chloride hydrochloride. The mixture is stirred for 1.5 hours and the organic solvent is distilled off under reduced pressure. The residual aqueous solution is placed in a chromatographic column of Diaion CHP20P and elution is carried out with water and 5% ethanol in that order. The fractions containing the desired product are collected and lyophilized to give 416 mg of sodium (3S,4S)-cis-3-[2-(2-chloroacetamidothiazol-4-yl)-(Z)-2-methoxyimino]-acetamido-4-succinimido-2-azetidinone-1-sulfonate.

$[\alpha]_D^{23}$ + 20.9° (c =0.505, $H_2O$)

IR spectrum $\nu_{max}^{KBr}$ $cm^{-1}$: 3480, 1785, 1710, 1280, 1055.

NMR spectrum ($D_2O$, 100MHz) δ: 2.88 (4H, s, $COCH_2CH_2CO$), 4.04 (3H, s, $OCH_3$), 4.44 (2H, s, $ClCH_2CO$), 5.81 (1H, d, J=5Hz, 3-H), 6.28 (1H, d, J=5Hz, 4-H), 7.04 (1H, s, thiazole 5-H).

Elemental analysis for $C_{15}H_{14}ClN_6NaO_9S_2 \cdot 2.5H_2O$:

Calcd.: (%) C 30.54; H 3.25; N 14.25

Found: (%) C 30.53; H, 3.36; N 14.17

(4) A mixture of 208 mg of sodium (3S,4S)-cis-3-[2-(2-chloroacetamidothiazol-4-yl)-(Z)-2-methoxyimino]-acetamido-4-succinimido-2-azetidinone-1-sulfonate, 150 mg of sodium methyldithiocarbamate, 3 drops of ethyl acetate and 7 ml of water is stirred at room temperature for 1 hour and 10 minutes, and then the mixture is shaken with ethyl aceate. The aqueous layer is separated and passed through a chromatographic column of Diaion CHP20P, elution being carried out with water. The fractions containing the desired product are collected and lyophilized to give 124 mg of the title compound.

$[\alpha]_D^{24}$ + 33.3° (c = 0.685, $H_2O$)

IR spectrum $\nu_{max}^{KBr}$ $cm^{-1}$: 3400, 1780, 1710, 1285, 1055.

NMR spectrum ($D_2O$, 100MHz) δ: 2.88 (4H, s, $COCH_2CH_2CO$), 4.00 (3H, s, $OCH_3$), 5.77 (1H, d, J=5Hz, 3-H), 6.26 (1H, d, J=5Hz, 4-H), 6.92 (1H, s, thiazole 5-H).

Elemental analysis, for $C_{13}H_{13}N_6NaO_8S_2 \cdot 2.5H_2O$:

Calcd.: (%) C, 30.41; H, 3.53; N, 16.37

Found: (%) C, 30.35; H, 3.49; N, 16.30

Example 2

Production of sodium (3S,4S)-cis-3-[2-(2-aminothiazol-4-yl)-(Z)-2-methoxyimino]acetamido-4-phthalimido-2-azetidinone-1-sulfonate

(1) To a solution of 1.5 g of phthalimide in 10 ml of dimethylformamide is added 10 ml of 1M sodium methoxide methanol and the mixture is stirred under cooling at −78°C. To the mixture is added 37 ml of a

dichloromethane solution containing 10 mMol of methyl 2-[(3R,4R)-trans-3-benzyloxycarboxamido-4-chloro-2-oxazetidin-1-yl]-2-oxoacetate and the resulting mixture is stirred for 20 minutes, followed by addition of 2 ml of 2N hydrochloric acid and 10 ml of methanol. The mixture is concentrated under reduced pressure and the residue is shaken with 50 ml of ethyl acetate and 10 ml of saturated aqueous sodium chloride. The organic layer is washed with water and concentrated. The residue is dissolved in 30 ml of dichloromethane and the solution is filtered. The filtrate is subjected to silica gel column chromatograhy using ethyl acetate-n-hexane (4:1) as the eluent. The fractions containing the desired product are collected and concentrated to dryness to give 1.86 g of (3S,4S)-cis-3-benzyloxycarboxamido-4-phthalimido-2-azetidinone.

mp 85 to 87°C

$[\alpha]_D^{20}$ + 63.2° (c = 0.435, $CH_3OH$)

IR spectrum $\nu_{max}^{KBr}$ $cm^{-1}$: 3350, 1780, 1715.

NMR spectrum ($CDCl_3$, 60NHz) δ: 4.97 (2H, s, —$CH_2O$), 5.69 (1H, dd, J=5 & 9Hz, 3-H), 5.98 (1H, d, J=5Hz, 4-H), 6.72 (1H, br, s, NH), 7.16 (5H, s, $C_6H_5$), 7.70 (4H, s, phthalyl).

Elemental analysis, for $C_{19}H_{15}N_3O_5 \cdot 1/3H_2O$:

Calcd.: (%) C, 61.45; H, 4.25; N, 11.32

Found: (%) C. 61.62; H, 4.17; N, 11.04

(2) A solution of 650 mg of (3S,4S)-cis-3-benzyloxycarboxamido-4-phthalimido-2-azetidinone in 1 ml of dimethylformamide is cooled to −50°C and a solution of 300 mg of sulfuric anhydride in 3 ml of dimethylformamide is added dropwise thereto. The mixture is allowed to stand at 5°C overnight and then stirred under ice-cooling, followed by addition of 1.2 g of sodium hydrogen carbonate. The resulting mixture is stirred and 40 ml of ether is added. The mixture is stirred and the supernatant is decanted off. To the residue is added 40 ml of ether and the decantation procedure is repeated. To the residue is added 20 ml of water and the mixture is stirred under ice-cooling for 10 minutes. The crystalline precipititate is collected by suction filtration, washed with ether and dried to give 660 mg of sodium (3S,4S)-cis-3-benzyloxycarboxamido-4-phthalimido-2-azetidinone-1-sulfonate.

mp 156 to 163°C

IR spectrum $\nu_{max}^{KBr}$ $cm^{-1}$: 3590, 3520, 3360, 1790, 1770, 1720, 1260, 1060.

NMR spectrum ($d_6$-DMSO, 100MHz) δ: 5.00 (2H, s, $CH_2O$), 5.40 (1H, dd, 5 & 9 Hz, 3-H), 6.05 (1H, d, J=5 & 9Hz, 4-H), 6.68 (1H, d, J=9Hz, NH), 7.26 (5H, s, $C_6H_5$), 7.93 (4H, s, phthalyl).

Elemental analysis, for $C_{19}H_{14}N_3NaO_8S \cdot 0.5H_2O$:

Calcd.: (%) 47.90; H, 3.17; N, 8.82

Found: (%) C, 47,70; H, 3.37; N, 8.79.

(3) A mixture of 610 mg of sodium (3S,4S)-cis-3-benzyloxycarboxamido-4-phthalimido-2-azetidinone-1-sulfonate, 0.5 g of 5% palladium-on-carbon and 5 ml of water is stirred under a hydrogen atmosphere at room temperature for 30 minutes. Methanol (5 ml) is added and the mixture is stirred under the same conditions for an hour. The reaction mixture is concentrated under reduced pressure and the methanol is distilled off. To the residue are added 0.5 g of 5% palladium-on-carbon and 5 ml of water and the mixture is stirred under a hydrogen atmosphere at room temperature for an hour. The reaction mixture is filtered and an adequate amount of water is added to the filtrate to make 15 ml. To this solution are added 0.8 g of sodium hydrogen carbonate and 5 ml of acetone, followed by dropwise addition of 0.5 g of 2-(2-chloroacetamidothiazol-4-yl)-(Z)-2-methoxyiminoacetyl chloride hydrochloride with stirring. The mixture is stirred at room temperature for an hour and then shaken with ethyl acetate (20 ml × 2). The aqueous layer is separated and placed in a chromatographic column of Diaion CHP-20P and the elution is carried out with water and 15% ethanol in that order. The fractions containing the desired product are collected and lyophilized to give 155 mg of sodium (3S,4S)-cis-[2-(2-chloroacetamidothiazol-4-yl)-(Z)-2-methoxyimino]acetamido-4-phthalimido-2-azetidinone-1-sulfonate.

IR spectrum $\nu_{max}^{KBr}$ $cm^{-1}$: 3430, 1780, 1720, 1260, 1040.

NMR spectrum ($D_6$-DMSO, 60MHz) δ: 3.11 (3H, s, $OCH_3$), 4.26 (2H, s, $ClCH_2CO$), 5.46 (1H, dd, J=5 & 8Hz, 3-H), 5.98 (1H, d, J=5Hz, 4-H), 7.20 (1H, s, thiazole 5-H), 7.23—7.90 (6H, m, 2 × NH and phthalyl).

Elemental analysis, for $C_{19}H_{14}ClN_6NaO_9S_2 \cdot 3H_2O$:

Calcd.: (%) C, 35.27; H, 3.12; N, 12.99

Found: (%) C, 35.52; H, 3.02; N, 13.20.

(4) A mixture of 125 mg of sodium (3S,4S)-cis-3-[2-(2-chloroacetamidothiazol-4-yl)-(Z)-2-methoxyimino]acetamido-4-phthalimido-2-azetidinone-1-sulfonate, 12 mg of sodium methyldithiocarbamate, 3 ml of water and 3 ml of acetone is stirred at room temperature for an hour. The solvent is distilled off under reduced pressure and the residue is shaken with ethyl acetate (5 ml × 2). The aqueous layer is separated and placed in a chromatographic column of Diaion CHP-20P and elution is carried out with water and 10% ethanol in that order. The fractions containing the desired product are collected and lyophilized to give 80 mg of the title compound.

$[\alpha]_D^{20}$ + 101.9° (c = 0.535, $CH_3OH$—$H_2O$ (3:1)).

IR spectrum $\nu_{max}^{KBr}$ $cm^{-1}$: 3400, 1780, 1720, 1280, 1050.

NMR ($d_6$-DMSO + $D_2O$, 60MHz) δ: 3.20 (3H, s, $OCH_3$), 5.64 (1H, d, J=5Hz, 3-H), 6.26 (1H, d, J=5Hz, 4-H), 6.65 (1H, s, thiazole 5-H), 7.86 (4H, s, phthalyl).

Elemental analysis, for $C_{17}H_{13}N_6NaO_8S_2{\cdot}1.5H_2O$:

Calcd.: (%) C, 37.57; H, 2.97; N, 15.46

Found: (%) C, 37.81; H, 3.19; N, 15.67.

Example 3

Production of sodium (3S,4S)-cis-3-[2-(2-aminothiazol-4-yl)-(Z)-2-methoxyimino]acetamido-4-glutarimido-2-azetidinone-1-sulfonate

(1) Methyl 2-[(3R,4R)-trans-3-benzyloxycarboxamido-4-chloro-2-oxoazetidin-1-yl]-2-oxoacetate is reacted with glutarimide in the same manner as Example 1-(1) to give (3S,4S)-cis-3-(benzyloxycarboxamido)-4-glutarimido-2-azetidinone.

$[\alpha]_D^{24}$ + 39.1° (c = 0.975, $CH_3OH$)

IR spectrum $\nu_{max}^{KBr}$ $cm^{-1}$: 3330, 1780, 1720, 1680.

NMR spectrum ($CDCl_3$, 90NHz) δ: 1.61—1.93 (2H, m, $CH\underline{CH_2}CH_2$), 2.45 — 2.66 (4H, m, 2 × $COCH_2$), 5.03 (2H, s, $OCH_2$), 5.31 (1H, dd, J=5 & 10Hz, 3-H), 5.72 (1H, d, J=$\overline{10}$Hz, NH), 6.21 (1H, d, J=5Hz, 4-H), 6.80 (1H, s, β-lactam NH), 7.29 (5H, s, $C_6H_5$).

(2) (3S,4S)-cis-(3-Benzyloxycarboxamido)-4-glutarimido-2-azetidinone is sulfonated in the same manner as Example 1-(2) to give sodium (3S,4S)-cis-3-(benzyloxycarboxamido)-4-glutarimido-2-azetidinone-1-sulfonate.

IR spectrum $\nu_{max}^{KBr}$ $cm^{-1}$: 3420, 1780, 1720, 1680, 1250, 1050.

(3) In the same manmner as Example 1-(3), sodium (3S,4S)-cis-3-(benzyloxycarboxamido)-4-glutarimido-2-azetidinone-1-sulfonate is subjected to a reductive reaction for elimination of protective group, followed by reaction with 2-(2-chloroacetamidothiazol-4-yl)-(Z)-2-methoxyiminoacetylchloride to give sodium (3S,4S)-3-[2-(2-chloroacetamidothiazol-4-yl)-(Z)-2-methoxyimino]acetamido-4-glutarimido-2-azetidinone-1-sulfonate.

IR spectrum $\nu_{max}^{KBr}$ $cm^{-1}$: 1780, 1690, 1250, 1050.

NMR spectrum ($d_6$-DMSO, 90MHz) δ: 1.70—2.70 (6H, m, —$(CH_2)3$—), 3.87 (3H, s, $OCH_3$), 4.34 (2H, s, $ClCH_2CO$), 5.49 (1H, dd, J=6 & 9Hz, 3-H), 6.40 (1H, d, J=6Hz, 4-H), 7.35 (1H, s, thiazole 5-H), 7.97 (1H, d, J=9Hz, NH).

(4) Sodium (3S,4S)-3-[2-(2-chloroacetamidothiazol-4-yl)-(Z)-2-methoxyimino)acetamido-4-glutarimido-2-azetidinone-1-sulfonate is subjected to a reaction for eliminating chloroacetyl group in the same manner as Example 1-(4) to give the title compound.

$[\alpha]_D^{25}$ + 44.7° (c = 0.465, $H_2O$)

IR spectrum $\nu_{max}^{KBr}$ $cm^{-1}$: 3380, 1780, 1690, 1250, 1050.

NMR spectrum ($D_2O$, 90MHz) δ: 1.85—2.90 (6H, m, —$(CH_2)_3$—), 4.00 (3H, s, $OCH_3$), 5.75 (1H, d, J=5.5Hz, 4-H), 6.79 (1H, d, J=5.5Hz, 3-H), 6.90 (1H, s, thiazole 5-H).

Elemental analysis, for $C_{14}H_{15}N_6NaO_8S_2{\cdot}2H_2O$:

Calcd.: (%) C, 32.43; H, 3.69; N, 16.21

Found: (%) C, 32.48; H, 3.91; N, 16.02

Example 4

Production of sodium (3S, 4RS)-3-[2-(2-aminothiazol-4-yl)-(Z)-2-methoxyimino]acetamido-4-(2,4-dioxo-thiazolidin-3-yl)-2-azetidinone-1-sulfonate

(1) A mixture of 203 mg of (3R,4R)-4-methylsulfonyl-3-tritylamino-2-azetidinone (E. G. Brain et al., J. C. S. Perkin I, *1976*, 447), 240 mg of thiazolidine-2,4-dione, 414 mg of potassium carbonate, 3 ml of dichloromethane, 1 ml of water and 30 mg of benzyltriethylammonium chloride is stirred at room temperature for 2 hours and then shaken with saturated aqueous sodium chloride. The organic layer is separated, dried and concentrated to dryness. The residue is dissolved in a small amount of methanol with warming and the solution is allowed to stand at room temperature. The resulting crystalline precipitate is collected by filtration, washed with methanol and n-hexane in that order, and dried to give 164 mg of (3S,4RS)-4-(2,4-dioxothiazolidin-3-yl)-3-tritylamino-2-azetidinone.

IR spectrum $\nu_{max}^{KBr}$ $cm^{-1}$: 3350, 1755, 1690.

NMR spectrum ($CDCl_3$, 90MHz) δ: 3.14 (½H, d, J=11Hz, NHTr (4R isomer)), 3.40 (½H, d, J=11Hz, NHTr (4S isomer)), 3.60 (1H, s, $SCH_2$ (4S isomer)), 3.74 (1H, s, $SCH_2$ (4R isomer)), 4.64 (½H, dd, J=5 & 11Hz, 3-H (4R isomer)), 4.88 (½H, d, J=2Hz, 4-H (4S isomer)), 5.13 (½H, dd, J=2 & 11Hz, 3-H (4S isomer)), 5.20 (½H, d, J=5Hz (4R isomer)), 7.00—7.50 (15H, m, trityl), 7.56 & 7.63 (each ½H, each s, β-lactam NH).

Elemental analysis, for $C_{25}H_{21}N_3O_3S{\cdot}O.2H_2O$:

Calcd.: (%) C, 67.16; H, 4.82: N, 9.40

Found: (%) C, 67.12; H, 4.87; N, 9,15

(2) A mixture of 164 mg of (3S,4RS)-4-thiazolidine-2,4-dion-3-yl)-3-tritylamino-2-azetidione, 150 mg of toluenesulfonic acid monohydrate and 15 ml of dichloromethane is stirred at room temperature for an hour and then concentrated to dryness. The residue is washed with ethyl acetate. A mixture of the residue thus treated, 6 ml of acetone, 210 mg of sodium hydrogen carbonate and 3 ml of water is stirred under ice-cooling and 165 mg of 2-(2-chloroacetamidothiazol-4-yl)-2-(Z)-methoxyiminoacetyl chloride hyrochloride is added dropwise thereto. The mixture is stirred for 30 minutes and extracted with 50 ml of ethyl acetate. The organic layer is washed with water, dried, and concentrated to dryness to give 220 mg of crude (3S,4RS)-3-[2-(2-chloroacetamidothiazol-4-yl)-(Z)-2-methoxyimino]acetamido-4-(thiazolidine-2,4-dion-3-yl)-2-azetidinone.

IR spectrum $\nu_{max}^{KBr}$ $cm^{-1}$: 3250, 1780, 1690.

(3) A mixture of 220 mg of (3S,4RS)-3-[2-(2-chloroacetamidothiazol-4-yl)-(Z)-2-methoxyimino]acetamido-4-(thiazolidine-2,4-dion-3-yl)-2-azetidinone, 1 ml of dimethylformamide and 4.5 ml of 10% sulfuric anhydridedimethylformamide is stirred at 5°C for 3 days. To the mixture is added 1.5 g of sodium hydrogen carbonate under cooling to −20°C and the temperature is raised to −15°C, and the mixture is stirred for 30 minutes. To the mixture is added ethyl ether, and the resulting powdery precipitate is collected by filtration, washed with ether and dissolved in 20 ml of ice water. The solution is placed in a chromatographic column of Diaion CHP20P and elution is carried out with water and 5% ethanol in that order. The fractions containing the desired product are collected and lyophilized to give 134 mg of sodium (3S,4RS)-3-[2-(2-chloroacetamidothiazol-4-yl)-(Z)-2-methoxyimino]acetamido-4-(thiazolidine-2,4-dione-3-yl)-2-azetidinone-1-sulfonate.

IR spectrum $\nu_{max}^{KBr}$ $cm^{-1}$: 3450, 1785, 1690, 1280, 1050.

(4) A mixture of 114 mg of sodium (3S,4RS)-3-[2-(2-chloroacetamidothiazol-4-yl)-(Z)-2-methoxyimino]acetamido-4-(thiazolidin-2,4-dion-3-yl)-2-azetidinone-1-sulfonate, 105 mg of sodium methyldithiocarbamate and 3 ml of water is stirred at room temperature for 30 minutes and then shaken with ethyl ether. The aqueous layer is passed through a chromatographic column of Diaion CHP20P, elution being carried out with water. The fractions containing the desired product are collected and lyophilized to give 63 mg of the title compound.

$[\alpha]_D^{24.5}$ + 9.2° (c = 0.51, $H_2O$)

IR spectrum $\nu_{max}^{KBr}$ $cm^{-1}$: 3420, 1780, 1690, 1280, 1050.

NMR spectrum ($d_6$-DMSO, 90MHz) δ: 3.85 (3H, s, $OCH_3$), 3,94—4.27 (2H, m, $COCH_2$), 5.40—5.97 (2H, m, 3-H & 4-H), 6.64 & 6.67 (1H, each s, thiazole 5-H), 7.06 (2H, br, $NH_2$), 8.28 (3/5H, d, J=7Hz, CONH (4S isomer)), 9.33 (2/5H, d, J=9Hz, CONH (4R isomer)).

Elemental analysis, for $C_{12}H_{11}N_6NaO_8S_3 \cdot 2.5H_2O$:

Calcd.: (%) C, 27.12; H, 3.03; N, 15.81

Found: (%) C, 27.03; H, 2.86; N, 15.68

## Example 5

Production of pyridinium (3S,4S)-cis-3-benzyloxycarboxamido-4-maleimido-2-azetidinone-1-sulfonate

(1) A mixture of 660 mg of maleimide and 6 ml of 1M sodium methoxide-methanol is stirred under cooling at −78°C and a solution of 2.18 g of 2-(methyl (3R,4R)-trans-3-benzyloxycarboxamido-4-chloro-2-oxoazetidin-1-yl)-2-oxoacetate in 22 ml of dichloromethane is added dropwise thereto. The mixture is stirred for 30 minutes and 0.5 ml of acetic acid, 30 ml of dichloromethane and 30 ml of saturated aqueous sodium chloride are added to the mixture in that order. The organic layer is separated and subjected to silica gel column chromatography using n-hexane-ethyl acetate (1:2) as the eluent. The fractions containing the desired product are collected and concentrated to dryness to give 940 mg of (3S,4S)-cis-3-benzyloxycarboxamido-4-maleimido-2-azetidinone.

mp 55 to 59°C

$[\alpha]_D^{22}$ + 19.0° (c = 0.505, $CHCl_3$)

IR spectrum $\nu_{max}^{KBr}$ $cm^{-1}$: 3330, 1780, 1710.

NMR spectrum ($CDCl_3$, 90MHz) δ: 5.05 (2H, s, $CH_2O$), 5.40—5.90 (3H, m, 4-H, 2-H & NH), 6.65 (2H, s, —CH=CH—), 6.70 (1H, s, NH), 7.30 (5H, s, $C_6H_5$).

Elemental analysis, for $C_{15}H_{13}N_3O_5 \cdot 0.2CH_3COOC_2H_5 \cdot 0.4H_2O$:

Calcd.: (%) C, 55.78; H, 4.56; N, 12.35

Found: (%) C, 55.92; H, 4.55; N, 12.36

(2) A mixture of 1 g of (3S,4S)-cis-3-benzyloxycarboxamido-4-maleimido-2-azetidinone, 5 ml of 10% sulfuric anhydride-dimethylformamide and 2 ml of dimethylformamide is stirred at −78°C for an hour and at 5°C overnight. The reaction mixture is cooled to −78°C and 2 ml of pyridine and ethyl ether are added in that order. The powder precipitate is collected by filtration, washed with ether and dried to give 576 mg of the title compound.

mp 135 to 137°C

IR spectrum $\nu_{max}^{KBr}$ $cm^{-1}$: 3390, 1770, 1720.

NMR spectrum ($d_6$-DMSO, 60MHz) δ: 5,12 (2H, s, $CH_2O$), 5.36 (1H, dd, J=5 & 9Hz, 3-H), 5.90 (1H, d, J=5Hz, 4-H), 6.10 (1H, br., $SO_3H$), 6.54 (1H, d, J=9Hz, NH), 7.08 (2H, s, —CH=CH—), 7.35 (5H, s, $C_6H_5$), 8.00—9.08 (5H, m, pyridine).

Example 6

Production of sodium (3S,4RS)-3-[2-(2-aminothiazol-4-yl)-(Z)-2-methoxyimino]acetamido-4-(4-ethyl-2,3-dioxo-1,4-piperazin-1-yl)-2-azetidinone-1-sulfonate

(1) 1-Ethyl-2,3-dioxo-1,4-piperazine (502 mg) is dissolved in 3 ml of 1M lithium methoxide-methanol and solution is added 90 ml of tetrahydrofuran, followed by rapid dropwise addition of a solution of 834 mg of (3R,4RS)-4-acetoxy-3-benzyloxycarboxamido-2-azetidinone in 10 ml of tetrahydrofuran cooled to −78°C. The mixture is stirred for 5 minutes and concentrated to dryness. The residue is chromatographed on a silica gel column (60 g, 3 × 20 cm), with elution being made rapidly with dichloromethane — ethyl acetate — methanol (2:2:1). The fractions containing the desired product are collected and concentrated to dryness and the residue is crystallized from dichloromethane-ethyl ether. The crystals are collected by filtration and dried to give 510 mg of (3S,4RS)-3-benzyloxycarboxamido-4-(4-ethyl-2,3-dioxo-1,4-piperazin-1-yl)-2-azetidinone.

mp 150 to 192°C (decompn.)

$[\alpha]_D^{21}$ −81.0° (c = 0.585, DMSO)

IR spectrum $\nu_{max}^{KBr}$ $cm^{-1}$: 3380, 1780, 1710, 1670, 1520, 1450, 1420, 1365, 1330, 1280, 1250, 1190, 1070.

NMR spectrum ($d_6$-DMSO, 100MHz) δ: 1.05 & 1.08 (3H, each t, J=7Hz, $\underline{CH_3}CH_2$), 3.2—3.7 (6H, m, $CH_3\underline{CH_2}$ & $\underline{NCH_2CH_2N}$), 4.62 (½H, dd, J=2 & 8Hz, 3-H (trans-isomer)), 4.98 (½H, J=5 & 8Hz, 3-H (cis-isomer)), 5.03 & 5.06 (2H, each s, $CH_2O$), 5.74 (½H, d, J=5Hz, 4-H (cis-isomer)), 5.82 (½H, d, J=2Hz, 4-H (trans-isomer)), 7.31 & 7.35 (5H, each s, $C_6H_5$), 7.86 — 8.02 (1H, each d, J=8Hz, CONH), 8.43 & 8.52 (1H, each br., β-lactam NH).

Elemental analysis, for $C_{17}H_{20}N_4O_5$:

Calcd.: (%) C, 56.64; H, 5.59; N, 15.54

Found: (%) C, 55.61; H, 5.75; N, 15.20.

(2) (3S,4RS)-3-Benzyloxycarboxamido-4-(4-ethyl-2,3-dioxo-1,4-piperazin-1-yl)-2-azetidinone (510 mg) is dissolved in 4 ml of dimethylformamide and, to the solution is added 10 ml of 10% sulfuric anhydride-dimethylformamide under cooling at −78°C. The mixture is allowed to stand at 5°C overnight. To the reaction mixture is added 4 g of sodium hydrogen carbonate and the mixture is stirred for 60 minutes, followed by addition of ethyl ether. The powder precipitates are collected by decantation and dissolved in 10 ml of ice water. The solution is passed through a chromatographic column (3 × 30 cm) of Diaion CHP-20P, the elution being carried out with water and 30% ethanol in that order. The fractions containing the desired product are collected and lyophilized to give 504 mg of sodium (3S,4RS)-3-benzyloxycarboxamido-4-(4-ethyl-2,3-dioxo-1,4-piperazin-1-yl)-2-azetidinone-1-sulfonate.

$[\alpha]_D^{21}$ −60.6° (c = 0.48, $H_2O$)

IR spectrum $\nu_{max}^{KBr}$ $cm^{-1}$: 3400, 2980, 1780, 1710, 1670, 1520, 1470, 1420, 1370, 1260, 1195, 1050, 1000, 750.

NMR spectrum ($D_2O$, 100MHz) δ: 1.11 & 1.18 (3H, t, J=7Hz, $\underline{CH_3}CH_2$), 2.9—3.9 (6H, m, $CH_3\underline{CH_2}$ & $NCH_2CH_2N$), 4.88 (½H, d, J=2Hz, 3-H (trans-isomer)), 5.16 (½H, d, J=5Hz (cis-isomer)), 5.15 (2H, s, $CH_2O$), 6.06 (½H, d, J=5Hz, 4-H (cis-isomer)), 6.20 (½H, d, J=2Hz, 4-H (trans-isomer)), 7.38 & 7.42 (5H, each s, $C_6H_5$).

Elemental analysis, for $C_{17}H_{19}N_4NaO_8S \cdot 1.5H_2O$:

Calcd.: (%) C, 41.72; H, 4.53; N, 11.45

Found: (%) C, 41.77; H, 4.66 N, 11.52.

(3) In the same manner as Example 1-(3), sodium (3S,4RS)-3-benzyloxycarboxamido-4-(4-ethyl-2,3-dioxo-1,4-piperazin-1-yl)-2-azetidinone-1-sulfonate is subjected to reductive reaction for elimination of protective group, followed by reaction with 2-(2-chloroacetamidothiazol-4-yl)-(Z)-2-methoxyiminoacetyl chloride to give sodium (3S,4RS)-3-[2-(2-chloroacetamidothiazol-4-yl)-(Z)-2-methoxyimino]acetamido-4-(4-ethyl-2,3-dioxo-1,4-piperazin-1-yl)-2-azetidinone-1-sulfonate.

$[\alpha]_D^{20}$ −76.8° (c = 0.69, $H_2O$)

IR spectrum $\nu_{max}^{KBr}$ $cm^{-1}$: 3400, 2950, 1780, 1680, 1550, 1470, 1370, 1280, 1200, 1040.

NMR spectrum ($D_2O$, 100MHz) δ: 1.20 & 1.23 (3H, each t, J=7Hz, $\underline{CH_3}CH_2$), 3.50 & 3.54 (2H, each q, J=7Hz, $CH_3\underline{CH_2}$), 3.80 (4H, br., s, $NCH_2CH_2N$), 3.93 & 4.05 (3H, each s, $O\underline{CH_3}$), 4.46 (2H, s, $ClCH_2$), 5.19 (½H, d, J=2Hz, 4-H (trans-isomer)), 5.46 (½H, d, J=5Hz, 4-H (cis-isomer)), 6.15 (½H, d, J=5Hz, 3-H (cis-isomer), 6.42 (½H, d, J=2Hz, 3-H (trans-isomer)), 7.46 & 7.53 (1H, each s, triazole 5-H).

Elemental analysis, for $C_{17}H_{19}ClN_7NaO_9S_2 \cdot 2.5H_2O$:

Calcd.: (%) C, 32.96; H, 3.82; N, 15.49

Found: (%) C, 32.47; H, 3.61; N, 15.28

(4) Sodium (3S,4RS)-3-[2-(2-chloroacetamidothiazol-4-yl)-(Z)-2-methoxyimino]acetamido-4-(4-ethyl-2,3-dioxo-1,4-piperazin-1-yl)-2-azetidinone-1-sulfonate is subjected to a reaction for elimination of chloroacetyl group in the same manner as Example 1-(4) to give the title compound.

$[\alpha]_D^{20}$ −78.0° (c = 0.5, $H_2O$)

IR spectrum $\nu_{max}^{KBr}$ $cm^{-1}$: 3450, 1780, 1670, 1540, 1470, 1375, 1280, 1200, 1060.

NMR spectrum ($D_2O$, 100MHz) δ: 1.21 (3H, t, J=7Hz, $\underline{CH_3}CH_2$), 3.52 & 3.54 (2H, each q, J=7Hz, $CH_3\underline{CH_2}$), 3.78 (4H, br., s, $NCH_2CH_2N$), 3.88 & 4.00 (3H, each s, $\underline{OCH_3}$), 5.16 (½H, d, J=2Hz, 4-H (trans-isomer)), 5.45 (½H, d, J=5Hz, 4-H (cis-isomer)), 6.14 (½H, d, J=5Hz, 3-H (cis-isomer)), 6.39 (½H, d, J=2Hz, 3-H (trans-isomer)), 6.90 & 7.00 (1H, each s, thiazole 5-H).

Elemental analysis, for $C_{15}H_{18}N_7NaO\ S_2 \cdot 2.5H_2O$:
Calcd.: (%) C, 32.37; H, 4.17; N, 17.62
Found: (%) C, 32.42; h, 4.00; N, 16.95

## Example 7

Production of sodium (3S,4S)-3-[2-(2-aminothiazol-4-yl)-(Z)-2-methoxyimino]acetamido-4-(imidazolidine-2,4-dion-3-yl)-2-azetidinone-1-sulfonate

(1) Methyl (3R,4R)-trans-2-(3-benzyloxycarboxamido-4-chloro-2-oxoazetidin-1-yl)-2-oxoacetate is reacted with imidazolidine-2,4-dione in the same manner as Example 1-(1) to give (3S,4S)-cis-3-benzyloxycarboxamido-4-(imidazolidine-2,4-dion-3-yl)-2-azetidinone.

mp 109 to 112°C
$[\alpha]_D^{23}$ +9.5° (c = 1.1, DMSO)
IR spectrum $\nu_{max}^{KBr}$ $cm^{-1}$: 3300, 1780, 1710.

NMR spectrum ($d_6$-DMSO, 60MHz) δ: 4.00 (2H, s, CO—$\underline{CH_2}$—$NH_2$—), 5.10 (2H, s, —$OCH_2$—), 5.35 (1H, d, d, J=5Hz, 10Hz, 3-H), 5.70 (1H, d, J=5Hz, 4-H), 6.60 (1H, d, J=10Hz, NH), 7.32 (5H, s, $C_6H_5$), 8.30 (2H, m, NH).

(2) In 30 ml of methanol is dissolved 1.8 g of (3S,4S)-cis-3-benzyloxycarboxamido-4-(imidazolidine-2,4-dion-3-yl)-2-azetidinone and catalytic reduction is carried out in the presence of 0.5 g of 10% palladium-on-carbon at room temperature and atmospheric pressure. After completion of the reaction, the reaction mixture is filtered and the filtrate is concentrated under reduced pressure. The residue is dissolved in 20 ml of tetrahydrofuran, and a solution of 850 mg of sodium hydrogen carbonate in 20 ml of water and 1.7 g of 2-(2-chloroacetamidothiazol-4-yl)-(Z)-2-methoxyiminoacetyl chloride hydrochloride are added. The mixture is stirred under ice-cooling for 30 minutes and then extracted with 100 ml of ethyl acetate. The extract is washed with water, dried over magnesium sulfate and concentrated under reduced pressure. The resulting crystalline precipitate is collected by filtration and dried to give 1.2 g of (3S,4S)-cis-3-[2-(2-chloroacetamidothiazol-4-yl)-(Z)-2-methoxyimino]acetamido-4-(imidazolidine-2,4-dion-3-yl)-2-azetidinone.

mp 168 to 170°C
$[\alpha]_D^{23}$ +40.8° (c = 0.78, DMSO)
IR spectrum $\nu_{max}^{KBr}$ $cm^{-1}$: 3300, 1760, 1720, 1680.

NMR spectrum ($d_6$-DMSO, 60MHz) δ: 3.90—4.00 (5H, m, $OCH_3$ & $CO\underline{CH_2}NH$), 4.30 (2H, s, $COCH_2Cl$), 5.35 (1H, m, 3-H), 5.70 (1H, d, J=5Hz, 4-H), 7.30 (1H, s, thiazole 5-H), 8.20 (2H, m, NH × 2).

(3) A solution of 500 mg of (3S,4S)-cis-3-[2-(2-chloroacetamidothiazol-4-yl)-(Z)-2-methoxyimino]acetamido-4-imidazolidine-2,4-dion-3-yl)-2-azetidinone in 2 ml of dimethylformamide is cooled to −70°C and a solution of 300 mg of sulfuric anhydride in 3 ml of dimethylformamide is added dropwise thereto. The mixture is allowed to stand in a refrigerator overnight and stirred at −70°C, followed by addition of 600 mg of sodium hydrogen carbonate. The resulting mixture is stirred and 20 ml of ether is added. After removal of the supernatant by decantation, 20 ml of ether is added to the residue and the decantation procedure is repeated. The residue is dissolved in 2 ml of water and the solution is subjected to chromatography on an XAD-2 column using water as the eluent. The fractions containing the desired product are collected and lyophilized to give 250 mg of sodium (3S,4S)-cis-3-[2-(2-chloroacetamidothiazol-4-yl)-(Z)-2-methoxyimino]acetamido-4-imidazolidine-2,4-dion-3-yl)-2-azetidinone-1-sulfonate.

$[\alpha]_D^{23}$ +32° (c = 0.56, $H_2O$)
IR spectrum $\nu_{max}^{KBr}$ $cm^{-1}$: 3480, 1785, 1710, 1280, 1055.

NMR spectrum ($D_2O$, 100MHz) δ: 4.00 (3H, s, $OCH_3$), 4.10—4.18 (2H, m, $CO\underline{CH_2}NH$), 4.34 (2H, s, $ClCH_2CO$), 5.60 (1H, d, J=5Hz, 3-H), 6.10 (1H, d, J=5Hz, 4-H), 7.01 (1H, s, thiazole-H).

Elemental analysis, for $C_{14}H_{13}N_7NaO_9S_2 \cdot 2H_2O$:
Calcd.: (%) C, 28.90; H, 3.25; N, 16.85
Found: (%) C, 28.94; H, 3.18; N, 16.76

(4) Sodium (3S,4S)-cis-3-[2-(2-chloroacetamidothiazol-4-yl)-(Z)-2-methoxyimino]acetamido-4-imidazolidine-2,4-dion-3-yl)-2-azetidinone-1-sulfonate is subjected to a reaction for elimination of chloroacetyl gorup in the same manner as Example 1-(4) to give the title compound.

$[\alpha]_D^{23}$ +25.8° (c = 0.4, $H_2O$)
IR spectrum $\nu_{max}^{KBr}$ $cm^{-1}$: 3380, 1780, 1690, 1250, 1050.

NMR spectrum ($D_2O$, 100MHz) δ: 4.00 (3H, s, $OCH_3$), 4.18 (1H, m, $CO\underline{CH_2}NH$), 5.70 (1H, d, J=5Hz, 3-H), 6.28 (1H, d, J=5Hz, 4-H), 6.94 (1H, s, thiazole 5-H).

Elemental analysis, for $C_{12}H_{12}N_7NaO_8S_2 \cdot 3H_2O$;
Calcd.: (%) C, 27.53; H, 3.47; N, 18.73
Found: (%) C, 27.45; H, 3.37; N, 18.51.

Example 8

Production of sodium (3S,4S)-3-[2-(2-aminothiazol-4-yl)-(Z)-2-methoxyimino]acetamido-4-(1-methylimidazolidine-2,4-dion-3-yl)-2-azetidinone-1-sulfonate

(1) 2-[(3R,4R)-trans-(3-Benzyloxycarboxamido-4-chloro-2-oxoazetidinon-1-yl)-2-oxoacetate is reacted with 3-methylimidazolin-2,4-dione in the same manner as Example 1-(1) to give (3S,4S)-cis-3-(benzyloxycarboxamido-4-(1-methylimidazolidine-2,4-dion-3-yl)-2-azetidinone.

mp 60 to 63°C

$[\alpha]_D^{25}$ −32.9° (c = 1.07, $CH_3OH$)

IR spectrum $\nu_{max}^{KBr}$ $cm^{-1}$: 3400, 1780, 1720, 1680.

NMR spectrum ($d_6$-DMSO, 60MHz) δ: 3.00 (3H, s, N—$CH_3$), 3.80 (2H, s, $COCH_2N$—), 5.00 (2H, s, $OCH_2$), 5.50 (1H, d, d, J=10 & 4.5Hz, 3-H), 5.80 (1H, d, J=4.5Hz, 4-H), 6.20 (1H, d, J=10Hz, NH), 7.30 (5H, s, $C_6H_5$), 7.80 (1H, s, NH).

(2) In the same manner as Example 7-(2), (3S,4S)-cis-3-(benzyloxycarboxamido-4-(1-methylimidazolidine-2,4-dion-3-yl)-2-azetidinone is subjected to reductive reaction for elimination protective group, followed by reaction with 2-(2-chloroacetamidothiazol-4-yl)-(Z)-2-methoxyiminoacetyl chloride to give (3S,4S)-cis-3-[2-(2-chloroacetamidothiazol-4-yl)-(Z)-2-methoxyimino]acetamido-4-(1-methylimidazolidine-2,4-dion-3-yl)-2-azetidinone.

mp 170 to 172°C

$[\alpha]_D^{22}$ +58.8° (c = 0.895, DMSO)

IR spectrum $\nu_{max}^{KBr}$ $cm^{-1}$: 3500, 1780, 1720, 1680.

NMR spectrum ($d_6$-DMSO, 60MHz) δ: 2.80 (3H, s, N—$CH_3$), 3.80 (5H, s, $OCH_3$ & $COCH_2N$), 4.35 (2H, s, $COCH_2Cl$), 5.40 (1H, d, d, J=10Hz & 5Hz, 3-H), 5.60 (1H, d, J=5Hz, 4-H), 7.33 (1H, s, thiazole 5-H).

(3) (3S,4S)-cis-3-[2-(2-Chloroacetamidothiazol-4-yl)-(Z)-2-methoxyimino]acetamido-4-(1-methylimidazolidine-2,4-dion-3-yl)-2-azetidinone is subjected to sulfonation in the same manner as Example 7-(3) to give sodium (3S,4S)-cis-3-[2-(2-chloroacetamidothiazol-4-yl)-(Z)-2-methoxyimino]acetamido-4-(1-methylimidazolidine-2,4-dion-3-yl)-2-azetidinone-1-sulfonate.

$[\alpha]_D^{22}$ +41.8° (c = 0.835, $H_2O$)

IR spectrum $\nu_{max}^{KBr}$ $cm^{-1}$: 3500, 1785, 1720, 1690, 1290, 1050.

NMR spectrum ($D_2O$, 90MHz) δ: 3.20 (3H, s, $NCH_3$), 4.20 (3H, s, $OCH_3$), 4.33 (2H, s, $COCH_2$ N—), 4.63 (2H, s, $ClCH_2CO$), 6.60 (1H, d, J=5Hz, 3-H), 6.43 (1H, d, J=5Hz, 4-H), 7.63 (1H, thiazole 5-H).

(4) Sodium (3S,4S)-cis-3-[2-(2-chloroacetamidothiazol-4-yl)-(Z)-2-methoxyimino]acetamido-4-(1-methylimidazolidine-2,4-dion-3-yl)-2-azetidinone-1-sulfonate is subjected to a reaction for eliminating chloroacetyl group in the same manner as Example 1-(4) to give the title compound.

$[\alpha]_D^{25}$ +55° (c = 0.915, $H_2O$)

IR spectrum $\nu_{max}^{KBr}$ $cm^{-1}$: 3450, 1780, 1720, 1680, 1290, 1250, 1050.

NMR spectrum ($D_2O$, 60MHz) δ: 3.02 (3H, s, $NCH_3$), 3.98 (3H, s, $OCH_3$), 4.16 (2H, s, $COCH_2$ N—), 5.84 (1H, d, J=5Hz, 3-H), 6.22 (1H, d, J=5Hz, 4-H), 6.90 (1H, s, thiazole 5-H).

Elemental analysis, for $C_{13}H_{14}N_7NaO_8S_2 \cdot 2.5H_2O$:

Calcd.: (%) C, 29.55; H, 3.62; N, 18.55

Found: (%) C, 29.86; H, 3.80; N, 18.59.

Example 9

Production of sodium (3S,4R)-trans-3-[2-(2-aminothiazol-4-yl)-(Z)-2-methoxyimino]acetamido-4-(1-methylimidazolidine-2,4-dion-3-yl)-2-azetidinone-1-sulfonate

(1) A solution of 3 g of methyl 2-(3R,4R)-trans-4-(chloro-3-phthalimido-2-oxoazetidin-1-yl)-2-oxoacetate [European laid open patent application No. 52,299] in 10 ml of dichloromethane is cooled to −78°C and, under stirring, a mixture of 1.5 g of 3-methylimidazolidine-2,4-dione and 8.5 ml of 1M sodium methoxide-methanol is added dropwise thereto. The resulting mixture is stirred for about 10 minutes, and to the mixture is added 10 ml of acetic acid. The resulting crystalline precipitate is collected by filtration, washed with water and dried to give 2.6 g of (3S,4R)-trans-4-(1-methylimidazolidine-2,4-dion-3-yl)-3-phthalimido-2-azetidinone.

mp 223 to 225°C

$[\alpha]_D^{25}$ −249.2° (c = 1.065, DMSO)

IR spectrum $\nu_{max}^{KBr}$ $cm^{-1}$: 3250, 1800, 1770, 1710.

NMR spectrum ($d_6$-DMSO, 60MHz) δ: 2.90 (3H, s, $NCH_3$), 4.00 (2H, s, $COCH_2$ N—), 5.80 (1H, d, J=2Hz), 6.30 (1H, s), 7.80 (4H, s, aromatic H), 8.80 (1H, s, NH).

(2) In 5 ml of dimethylformamide is dissolved 2.0 g of (3S,4R)-trans-4-(1-methylimidazolidine-2,4-dion-3-yl)-3-phthalimido-2-azetidinone, and to the mixture are added 2 ml of triethylamine and 2.7 g of tert-

butyldimethylsilyl chloride. The mixture is stirred under ice-cooling for an hour and then poured into 20 ml of ice water. The mixture is extracted with 100 ml of ethyl acetate and the extract is washed with water and dried over anhydrous magnesium sulfate. The solvent is then distilled off under reduced pressure and the residue is subjected to silica gel column chromatography using ethyl acetate-n-hexane (2:1) as the eluent to give 2.0 g of (3S,4S)-trans-1-tert-butyldimethylsilyl-4-(1-methylimidazolidine-2,4-dion-3-yl)-3-phthalimido-2-azetidinone.

mp 100 to 103°C

$[\alpha]_D^{25}$ −188.7° (c = 1.045, $CH_3OH$)

IR spectrum $\nu_{max}^{KBr}$ $cm^{-1}$: 2900, 1780, 1770, 1720.

NMR spectrum ($d_6$-DMSO, 60MHz) δ: 0.90 (3H, s, $CH_3$), 0.23 (3H, s, $CH_3$), 0.90 (9H, s, $C(CH_3)_3$), 2.86 (3H, s, $NCH_3$), 4.00 (2H, s, $COCH_2N$—), 5.80 (1H, d, J=2Hz), 6.23 (1H, d, J=2Hz), 7.80 (4H, s, aromatic H).

(3) To 2.0 g of (3S,4R)-trans-1-tert-butyldimethylsilyl-4-(1-methylimidazolidine-2,4-dion-3-yl)-3-phthalimido-2-azetidinone is added 14 ml of 0.1 M methylhydrazinedimethoxyethane and the mixture is allowed to stand at room temperature for an hour and then concentrated under reduced pressure. The residue is dissolved in 20 ml of dichloromethane and the solution is allowed to stand at room temperature overnight. The crystalline precipitate is filtered off and the filtrate is concentrated under reduced pressure. The residue is dissolved in 20 ml of tetrahydrofuran and, under ice-cooling, a solution of 700 mg of sodium hydrogen carbonate in 20 ml of water and 1.3 g of 2-(2-chloroacetamidothiazol-4-yl)-(Z)-2-methoxyiminoacetyl chloride hydrochloride are added. The resulting mixture is stirred for 30 minutes and then extracted with 100 ml of ethyl acetate. The extract is washed with water and dried. The solvent is then distilled off under reduced pressure and the residue is subjected to silica gel column chromatography using ethyl acetate as the eluent to give 1.8 g of (3S,4R)-/-tert-butyldimethylsilyl-3-[2-(2-chloroacetamidothiazol-4-yl)-(Z)-2-methoxyimino]acetamido-4-(1-methylimidazolidine-2,4-dion-3-yl)-2-azetidinone. The product is dissolved in 30 ml of methanol and 150 mg of potassium fluoride is added. The mixture is stirred under ice-cooling for 3 hours and then concentratd to about half of its original volume under reduced pressure. To the concentrate are added 20 ml of water and 200 ml of ethyl acetate and the organic layer is separated, washed with water, dried and concentrated under reduced pressure to give 0.65 g of (3S,4R)-trans-3-[2-(2-chloroacetamidothiazol-4-yl)-(Z)-2-methoxyimino]acetamido-4-(1-methylimidazolidine-2,4-dion-3-yl)-2-azetidinone.

mp 213 to 215°C

$[\alpha]_D^{25}$ −73.2° (c = 0.9, DMSO)

IR spectrum $\nu_{max}^{KBr}$ $cm^{-1}$: 3200, 1780, 1710, 1680.

NMR spectrum ($d_6$-DMSO, 60MHz) δ: 2.82 (3H, s, $NCH_3$), 3.80 (3H, s, $OCH_3$) 4.00 (2H, s, $COCH_2N$), 4.30 (2H, s, $COCH_2Cl$), 5.36 (1H, d, J=2Hz, 4-H), 5.68 (1H, d, d, J=10Hz & 2Hz, 3-H), 7.30 (1H, s, thiazole 5-H).

(4) (3S,4R)-trans-3-[2-(2-Chloroacetamidothiazol-4-yl)-(Z)-2-methoxyimino]acetamido-4-(1-methylimidazolidine-2,4-dion-3-yl)-2-azetidinone is subjected to sulfonation in the same manner as Example 7-(3) to give sodium (3S,4R)-trans-3-[2-(2-chloroacetamidothiazol-4-yl)-(Z)-2-methoxyimino]acetamido-4-(1-methylimidazolidine-2,4-dion-3-yl)-2-azetidinone-1-sulfonate.

$[\alpha]_D^{22}$ −57.1° (c = 0.83, $H_2O$)

IR spectrum $\nu_{max}^{KBr}$ $cm^{-1}$: 3400, 1780, 1720, 1670, 1280, 1050.

NMR spectrum ($D_2O$, 90MHz) δ: 3.20 (3H, s, N—$CH_3$), 4.23 (3H, s, $OCH_3$), 4.33 (2H, s, $COCH_2N$—), 4.60 (2H, s, $ClCH_2CO$), 6.00 (1H, d, J=2Hz, 4-H), 6.20 (1H, d, J=2Hz, 3-H), 7.63 (1H, s, thiazole 5-H).

(5) (3S,4R)-trans-3-[2-(2-Chloroacetamidothiazol-4-yl)-(Z)-2-methoxyimino]acetamido-4-(1-methylimidazolidine-2,4-dion-3-yl)-2-azetidinone is subjected to a reaction for eliminating chloroacetyl group in the same manner as Example 1-(4) to give the title compound.

$[\alpha]_D^{25}$ −47.3° (c = 0.965, $H_2O$)

IR spectrum $\nu_{max}^{KBr}$ $cm^{-1}$: 3450, 1780, 1720, 1680, 1280, 1050.

NMR spectrum ($D_2O$, 100MHz) δ: 3.02 (3H, s, $NCH_3$), 4.00 (3H, s, $OCH_3$), 4.16 (2H, s, $COCH_2N$—), 5.78 (1H, d, J=2Hz, 3-H), 5.98 (1H, d, J=2Hz, 4-H), 6.96 (1H, s, thiazole 5-H).

Elemental analysis, for $C_{13}H_{14}N_7NaO_8S_2 \cdot 2H_2O$:

Calcd.: (%) C, 30.06; H, 3.49; N, 18.88

Found: (%) C, 30.31; H, 3.80; N, 18.63

Example 10

Production of sodium (3S,4S)-cis-3-[2-(2-aminothiazol-4-yl)-(Z)-2-methoxyimino]acetamido-4-(5-methyloxazolidine-2,4-dione-3-yl)-2-azetidinone-1-sulfonate

(1) 2-[(3R,4R)-trans-(3-Benzyloxycarboxamido-4-chloro-2-azetidin-1-yl)]-2-oxoacetate is reacted with 5-methyloxazolidine-2,4-dione in the same manner as Example 1-(1) to give (3S,4S)-cis-3-benzyloxycarboxamido-4-(5-methyloxazolidine-2,4-dion-3-yl)-2-azetidinone.

mp 167 to 170°C

$[\alpha]_D^{25}$ −10° (c = 0.82, DMSO)
IR spectrum $\nu_{max}^{KBr}$ $cm^{-1}$: 1830, 1760, 1730, 1700.
NMR spectrum ($CDCl_3$, 60MHz) δ: 1.46 (3H, d, J=7Hz, $CH_3$), 4.90 (1H, m), 5.00 (2H, s, $OCH_2$), 5.30 (1H, d, d, J=4.5Hz & 10Hz, 3-H), 5.50 (1H, d, J=4.5Hz, 4-H), 6.80 (1H, d, J=10Hz, CONH), 7.30 (5H, s, $C_6H_5$), 7.90 (1H, br., NH).

(2) In the same manner as Example 7-(2), (3S,4S)-cis-3-benzyloxycarboxamido-4-(5-methyloxazolidine-2,4-dion-3-yl)-2-azetidinone is subjected to reductive reaction for elimination of protective group, followed by reaction with 2-(2-chloroacetamidothiazol-4-yl)-(Z)-2-methoxyiminoacetyl chloride to give (3S,4S)-cis-3-[2-(2-chloroacetamidothiazol-4-yl)-(Z)-2-methoxyimino]acetamido-4-(5-methyloxazolidine-2,4-dion-3-yl)-2-azetidinone.
mp 240 to 242°C
IR spectrum $\nu_{max}^{KBr}$ $cm^{-1}$: 3250, 1810, 1780, 1745, 1695.
NMR spectrum ($d_6$-DMSO, 60MHz) δ: 1.40 (3H, d, J=7Hz, $CH_3$), 3.80 (3H, s, $OCH_3$), 4.53 (2H, s, $COCH_2Cl$), 4.96 (1H, q, J=7Hz, $-\overset{|}{\underline{C}\underline{H}}CH_3$), 5.40 (1H, d, d, J=10Hz & 5Hz, 3-H), 5.60 (1H, d, J=5Hz, 4-H), 7.36 (1H, s, thiazole 5-H).

(3) (3S,4S)-cis-3-[2-(2-Chloroacetamidothiazol-4-yl)-(Z)-2-methoxyimino]acetamido-4-(5-methyloxazolidine-2,4-dion-3-yl)-2-azetidinone is subjected to sulfonation in the same manner as Example 7-(3) to give sodium (3S,4S)-cis-3-[2-(2-chloroacetamidothiazol-4-yl)-(Z)-2-methoxyimino]acetamido-4-(5-methyloxazolidine-2,4-dion-3-yl)-2-azetidinone-1-sulfonate.
$[\alpha]_D^{25}$ +9.2° (c = 1.055, $H_2O$)
IR spectrum $\nu_{max}^{KBr}$ $cm^{-1}$: 3500, 1790, 1750, 1680, 1280, 1050.

(4) Sodium (3S,4S)-cis-3-[2-(2-chloroacetamidothiazol-4-yl)-(Z)-2-methoxyimino]acetamido-4-(5-methyloxazolidine-2,4-dion-3-yl)-2-azetidinone-1-sulfonate is subjected to a reaction for eliminating chloroacetyl group in the same manner as Example 1-(4) to give sodium (3S,4S)-3-[2-(2-aminothiazol-4-yl)-(Z)-2-methoxyimino]acetamido-4-(5-methyloxazolidine-2,4-dion-3-yl)-2-azetidinone-1-sulfonate.
$[\alpha]_D^{25}$ +20.7° (c = 0.97, $H_2O$)
IR spectrum $\nu_{max}^{KBr}$ $cm^{-1}$: 3450, 1810, 1785, 1745, 1670, 1620, 1280, 1050.
NMR spectrum ($D_2O$, 100MHz) δ: 1.60 (3H, d, J=7.5Hz, $CH_3$). 4.00 (3H, s, $OCH_3$), 5.22 (1H, t, J=7.5Hz,

$$-\overset{\underline{H}}{\underset{|}{\overset{|}{C}}}-CH_3),$$

5.75 (1H, d, J=5Hz, 3-H), 6.22 (1H, d, J=5Hz, 4-H), 6.95 (1H, s, thiazole 5-H).
Elemental analysis, for $C_{13}H_{13}N_6NaO_9S_2 \cdot 2H_2O$:
Calcd.: (%) C, 30.00; H, 3.92; N, 16.15
Found: (%) C, 30.06; H, 3.50; N, 16.00.

## Example 11

Production of sodium (3S,4S)-cis-3-[2-(2-aminothiazol-4-yl)-(Z)-2-(1-carboxy-1-methylethoxyimino)]-acetamido-4-succinimido-2-azetidinone-1-sulfonate

(1) In the same manner as Example 7-(2), (3S,4S)-cis-3-benzyloxycarboxamido-4-succinimido-2-azetidinone is subjected to a reductive reaction for elimination of protective group, followed by reaction with 2-(2-chloroacetamidothiazol-4-yl)-(Z)-2-(1-p-nitrobenzyloxycarbonyl-1-methylethoxyimino]acetyl chloride to give (3S,4S)-cis-3-[2-(2-chloroacetamidothiazol-4-yl)-(Z)-2-(1-p-nitrobenzyloxycarbonyl-1-methylethoxyimino)]-acetamido-4-succinimido-2-azetidinone.
mp 162 to 165°C
$[\alpha]_D^{25}$ +6.9° (c = 1.05, DMSO)
IR spectrum $\nu_{max}^{KBr}$ $cm^{-1}$: 3290, 1770, 1710, 1680.
NMR spectrum ($d_6$-DMSO, 60MHz) δ: 2.60 (4H, s, $COCH_2CH_2CO$), 4.33 (2H, s, $COCH_2Cl$), 4.80 (2H, s, $CH_2COO$), 5.26 (2H, s, $OCH_2-$), 5.60—5.80 (2H, m, 3-H and 4-H), 7.23 (1H, s, thiazole 5-H), 7.50—7.70 and 8.00—8.20 (4H, m, aromatic H).

(2) (3S,4S)-cis-3-[2-(2-Chloroacetamidothiazol-4-yl)-(Z)-2-(1-p-nitrobenzyloxycarbonyl-1-methylethoxyimino)]-acetamido-4-succinimido-2-azetidinone is subjected to sulfonation in the same manner as Example 7-(3) to give sodium (3S,4S)-cis-3-[2-(2-chloroacetamidothiazol-4-yl)-(Z)-2-(1-p-nitrobenzyloxycarbonyl-1-methylethoxyimino)]-acetamido-4-succinimido-2-azetidinone-1-sulfonate.
$[\alpha]_D^{25}$ +4.2° (c = 0.95, $H_2O$)
IR spectrum $\nu_{max}^{KBr}$ $cm^{-1}$: 3340, 1780, 1710, 1280, 1050.

NMR spectrum ($d_6$-DMSO, 60MHz) δ: 1.53 (6H, s, —C—$(CH_3)_2$), 2.60 (4H, s, $COCH_2CH_2CO$), 3.20 (3H, s, $OCH_3$), 4.33 (2H, s, $COCH_2$ N—), 5.30 (2H, s, $OCH_2$), 5.60 (1H, d, d, J=10Hz and 5Hz), 5.90 (1H, d, J=10Hz), 7.33 (1H, s, thiazole 5-H).

(3) In the same manner as Example 1-(4), sodium (3S,4S)-cis-3-[2-(2-chloroacetamidothiazol-4-yl)-(Z)-2-(1-p-nitrobenzyloxycarbonyl-1-methylethoxyimino]acetamido-4-succinimido-2-azetidinone-1-sulfonate is subjected to a reaction for eliminating chloroacetyl group and subjected to a reductive reaction for elimination of protective group to give sodium (3S,4S)-cis-3-[2-(2-aminothiazol-4-yl)-(Z)-2-(1-carboxy-1-methylethoxyimino)]acetamido-4-succinimido-2-azetidinone-1-sulfonate.

$[\alpha]_D^{25}$ −1.1° (c = 0.86, $H_2O$)

IR spectrum $\nu_{max}^{KBr}$ $cm^{-1}$: 3400, 1780, 1705, 1670, 1280, 1050.

NMR spectrum ($D_2O$, 60MHz) δ: 1.50 (6H, s, $CH_3 \times 2$), 2.80 (4H, s, $COCH_2CH_2CO$), 5.94 (1H, d, J=5Hz, 3-H), 6.25 (1H, d, J=5Hz), 6.90 (1H, s, thiazole 5-H).

Elemental analysis, for $C_{16}H_{17}N_6NaO_{10}S_2 \cdot H_2O$:

Calcd.: (%) C, 31.37; H, 4.11; N, 13.72

Found: (%) C, 31.47; H, 4.14; N, 13.46

Example 12

Production of sodium (3S,4S)-cis-3-[2-(2-aminothiazol-4-yl)-(Z)-2-(1-carboxymethoxyimino)]acetamido-4-succinimido-2-azetidinone-1-sulfonate

(1) In the same manner as Example 7-(2), (3S,4S)-cis-3-benzyloxycarboxamido-4-succinimido-2-azetidinone is subjected to reductive reaction for elimination of protective group, followed by reaction with 2-(2-chloroacetamidothiazol-4-yl)-(Z)-2-(p-nitrobenzyloxycarbonylmethoxyimino)acetyl chloride to give (3S,4S)-cis-3-[2-(2-chloroacetamidothiazol-4-yl)-(Z)-2-(p-nitrobenzyloxycarbonylmethoxyimino)]-acetamido-p-succinimido-2-azetidinone.

mp 275 to 277°C

$[\alpha]_D^{25}$ +25.2° (c = 0.93, DMSO)

IR spectrum $\nu_{max}^{KBr}$ $cm^{-1}$: 3270, 1765, 1710, 1680.

NMR spectrum ($d_6$-DMSO, 60MHz) δ: 1.50 (6H, s, $CH_3 \times 2$), 2.56 (4H, s, $COCH_2CH_2CO$), 4.30 (3H, s, $COCH_2Cl$), 5.26 (2H, s, $OCH_2$), 5.60—5.80 (2H, m, 3-H, 4-H), 7.50—7.70 and 8.00—8.20 (4H, m, aromatic -H).

(2) (3S,4S)-cis-3-[2-(2-Chloroacetamidothiazol-4-yl)-(Z)-2-(p-nitrobenzyloxycarbonylmethoxy]-acetamido-4-succinimido-2-azetidinone is subjected to sulfonation in the same manner as in Example 7-(3) to give sodium (3S,4S)-cis-3-[2-(2-chloroacetamidothiazol-4-yl)-(Z)-2-(p-nitrobenzyloxycarbonyl-methoxyimino)]acetamido-4-succinimido-2-azetidinone-1-sulfonate.

$[\alpha]_D^{25}$ +9.2° (c = 0.595, $H_2O$)

IR spectrum $\nu_{max}^{KBr}$ $cm^{-1}$: 3400, 1780, 1710, 1680, 1280, 1050.

NMR spectrum ($d_6$-DMSO, 60MHz) δ: 2,60 (4H, s, $COCH_2CH_2CO$), 3.20 (3H, s, $OCH_3$), 4.30 (2H, s, —$COCH_2Cl$), 4.80 (2H, s, —$CH_2COO$), 5.36 (2H, s, $OCH_2$—), 5.53 (1H, d, d, J=10Hz and 5Hz, 3-H), 5.83 (1H, d, J=5Hz, 4-H), 7.36 (1H, s, thiazole 5-H), 7.50—7.70 and 8.03—8.20 (4H, m, aromatic H).

(3) In the same manner as Example 1-(4) sodium (3S,4S)-cis-3-[2-(2-chloroacetamidothiazol-4-yl)-(Z)-2-(p-nitrobenzyloxycarbonylmethoxyimino)]acetamido-4-succinimido-2-azetidinone-1-sulfonate is subjected to a reaction for eliminating chloroacetyl group and subjected to a reductive reaction for elimination of protective group to give sodium (3S,4S)-cis-3-[2-(2-aminothiazol-4-yl)-(Z)-2-(1-carboxymethoxy-imino)]acetamido-4-succinimido-2-azetidinone-1-sulfonate.

$[\alpha]_D^{25}$ +18.9° (c = 0.98, $H_2O$)

IR spectrum $\nu_{max}^{KBr}$ $cm^{-1}$: 3400, 1780, 1710, 1680, 1280, 1050.

NMR spectrum ($D_2O$, 100MHz) δ: 2.86 (4H, s, $COCH_2CH_2CO$), 4.58 (2H, s, $OCH_2$), 5.82 (1H, d, J=5Hz, 3-H), 6.26 (1H, d, J=5Hz, 4-H), 6.98 (1H, s, thiazole 5-H).

Example 13

250 mg (potency) each portion of sodium (3S,4S)-cis-3-[2-(2-aminothiazol-4-yl)-(Z)-2-methoxyimino]acetamido-4-succinimido-2-azetidinone-1-sulfonate is distributed into sterile vials each being of 12 ml capacity, which are then sealed under reduced pressure (50 mmHg).

Example 14

250 mg (potency) each portion of sodium (3S,4S)-3-[2-(2-aminothiazol-4-yl)-(Z)-2-methoxyimino]acetamido-4-(2,4-dioxo-1-methylimidazolidin-3-yl)-2-azetidinone-1-sulfonate (500 mg) is distributed into vials, each being of 17 ml capacity, which are then sealed.

**Claims**

1. A 1-sulfo-2-azetidinone derivative of the formula:

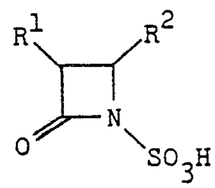

wherein $R^1$ is an amino group which may optionally be acylated with an acyl group commonly employed in β-lactam antibiotics or protected;

$R^2$ is a five- or six-membered cyclic group represented by the formula:

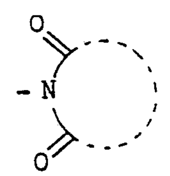

or

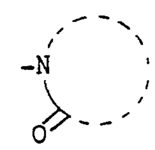

wherein the dotted arc portion may contain at least one of nitrogen, sulfur and oxygen as an arc-constituting member the dotted arc portion being optionally condensed with a benzene ring, and the hydrogen atom or atoms on the dotted arc portion being optionally substituted by an alkyl group containing 1 to 4 carbon atoms, a sulfo group or an oxo group; or a pharmaceutically acceptable salt thereof.

2. A compound as claimed in Claim 1, namely (3S,4S)-cis-3-[2-(2-aminothiazol-4-yl)-(Z)-2-methoxyimino]acetamido-4-succinimido-2-azetidinone-1-sulfonic acid, or a pharmaceutically acceptable salt thereof.

3. A compound as claimed in Claim 1, namely (3S,4S)-3-[2-(2-aminothiazol-4-yl)-(Z)-2-methoxyimino]acetamido-4-(2,4-dioxoimidazolidin-3-yl)-2-azetidinone-1-sulfonic acid, or a pharmaceutically acceptable salt thereof.

4. A compound as claimed in Claim 1, namely (3S,4S)-3-[2-(2-aminothiazol-4-yl)-(Z)-2-methoxyimino]acetamido-4-(1-methyl-2,4-dioxoimidazolidin-3-yl)-2-azetidinone-1-sulfonic acid, or a pharmaceutically acceptable salt thereof.

5. A compound as claimed in Claim 1, namely (3S,4S)-3-[2-(2-aminothiazol-4-yl)-(Z)-2-(1-carboxy-methoxyimino)]acetamido-4-succinimido-2-acetidinone-1-sulfonic acid, or a pharmaceutically acceptable salt thereof.

6. A method of producing a compound of claim 1, which comprises sulfonating a 2-azetidinone derivative of the formula:

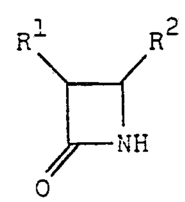

wherein the symbols are as defined in claim 1, or a salt or silyl derivative thereof.

7. A method of producing a compound of claim 1, wherein $R^1$ is an acylated amino group; and $R^2$ is as defined in claim 1, which comprises acylating a 3-amino-2-azetidinone derivative of the formula:

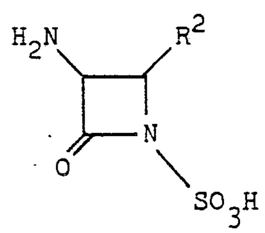

wherein $R^2$ is as defined in claim 1, or a salt or silyl derivative thereof.

8. A pharmaceutical composition comprising a compound of claim 1, wherein $R^1$ is an acylated amino group; and $R^2$ is as defined in claim 1, as an effective ingredient.

**Patentansprüche**

1. 1-Sulfo-2-azetidinon-Derivat der Formel

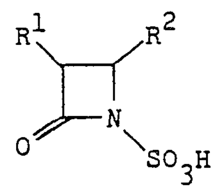

in der

$R^1$ eine Amino-Gruppe ist, die gegebenenfalls mit einer Acyl-Gruppe, die allgemein in β-Lactam-Antibiotika verwendet wird, acyliert oder geschützt sein kann, und

$R^2$ eine fünf- oder sechsgliedrige cyclische Gruppe der Formeln

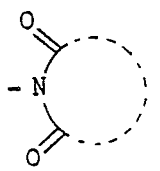

oder

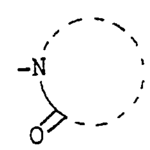

ist, worin der punktierte Bogen-Teil wenigstens ein Atom des Stickstoffs, Schwefels und Sauerstoffs als bogenbildendes Element enthalten kann, wobei der punktierte Bogen-Teil gegebenenfalls mit einem Benzol-Ring kondensiert ist und die Wasserstoff-Atome oder Atome an dem punktierten Bogen-Teil gegebenenfalls durch eine Alkyl-Gruppe mit 1 bis 4 Kohlenstoff-Atomen, eine Sulfo-Gruppe oder eine Oxo-Gruppe substituiert sind, oder ein pharmazeutisch annehmbares Salz desselben.

2. Verbindung nach Anspruch 1, nämlich (3S,4S)-3-[2-(2-Aminothiazol-4-yl)-(Z)-2-methoxyimino]acetamido-4-succinimido-2-azetidinon-1-sulfonsäure, oder ein pharmazeutisch annehmbares Salz derselben.

3. Verbindung nach Anspruch 1, nämlich (3S,4S)-3-[2-(2-Aminothiazol-4-yl)-(Z)-2-methoxyimino]acetamido-4-(2,4-dioxoimidazolidin-3-yl)-2-azetidinon-1-sulfonsäure, oder ein pharmazeutisch annehmbares Salz derselben.

4. Verbindung nach Anspruch 1, nämlich (3S,4S)-3-[2-(2-Aminothiazol-4-yl)-(Z)-2-methoxyimino]acetamido-4-(1-methyl-2,4-dioxoimidazolidin-3-yl)-2-azetidinon-1-sulfonsäure, oder ein pharmazeutisch annehmbares Salz derselben.

5. Verbindung nach Anspruch 1, nämlich (3S,4S)-3-[2-(2-Aminothiazol-4-yl)-(Z)-2-(1-carboxy-methoxyimino)]acetamido-4-succinimido-2-azetidinon-1-sulfonsäure, oder ein pharmazeutisch annehmbares Salz derselben.

6. Verfahren zur Herstellung einer Verbindung nach Anspruch 1, dadurch gekennzeichnet, daß ein 2-Azetidinon-Derivat der Formel

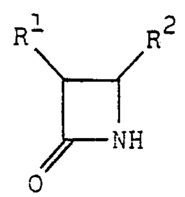

in der die Symbole die in Anspruch 1 angegebenen Bedeutungen haben, oder ein Salz oder Silyl-Derivat derselben sulfoniert wird.

7. Verfahren zur Herstellung einer Verbindung nach Anspruch 1, in der $R^1$ eine acylierte Amino-Gruppe ist und $R^2$ die in Anspruch 1 angegebenen Bedeutungen hat, dadurch gekennzeichnet, daß ein 3-Amino-2-azetidinon-Derivat der Formel

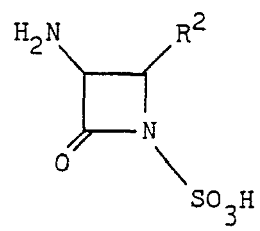

in der $R^2$ die in Anspruch 1 angegebenen Bedeutungen hat, oder ein Salz oder Silyl-Derivat derselben acyliert wird.

8. Pharmazeutische Zusammensetzung, enthaltend eine Verbindung nach Anspruch 1, in der $R^1$ eine acylierte Amino-Gruppe ist und $R^2$ die in Anspruch 1 angegebenen Bedeutungen hat, als Wirkstoff.

**Revendications**

1. Dérivé 1-sulfo-2-azétidinone, de formule:

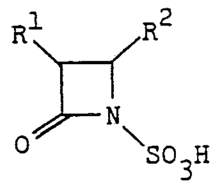

dans laquelle $R^1$ est un groupe amino, qui peut être éventuellement acylé avec un groupe acyle utilisé habituellement dans les antibiotiques β-lactames, ou protégé;

$R^2$ est un groupe cyclique à cinq ou six chaînons, représenté par la formule:

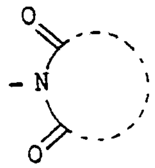

ou

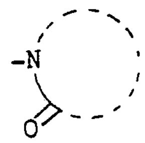

dans laquelle la portion de cercle représentée en pointillés peut contenir au moins un atome de soufre, d'oxygène, ou d'azote, en tant qu'élément constitutif du cycle, la portion de cercle représentée en pointillés étant éventuellement condensée avec un noyau benzénique, et le ou les atomes d'hydrogène dans la partie de cercle représentée en pointillé étant éventuellement substitués par un groupe contenant de 1 à 4 atomes de carbone, un groupe sulfo ou un groupe oxo; ou un sel pharmaceutiquement acceptable de ce dérivé.

2. Composé conforme à la revendication 1, à savoir l'acide (3S,4S)-3-[2-(2-aminothiazol-4-yl)-(Z)-2-méthoxyimino]acétamido-4-succinimido-2-azétidinone-1-sulfonique, ou un sel pharmaceutiquement acceptable de ce composé.

3. Composé conforme à la revendication 1, à savoir l'acide (3S,4S)-3-[2-(2-aminothiazol-4-yl)-(Z)-2-méthoxyimino]acétamido-4-(2,4-dioxoimidazolidine-3-yl)-2-azétidinone-1-sulfonique, ou un sel pharmaceutiquement acceptable de ce composé.

4. Composé conforme à la revendication 1, à savoir l'acide (3S,4S)-3-[2-(2-aminothiazol-4-yl)-(Z)-2-méthoxyimino]acétamido-4-(1-méthyl-2,4-dioxoimidazolidine-3-yl)-2-azétidinone-1-sulfonique, ou un sel pharmaceutiquement acceptable de ce composé.

5. Composé conforme à la revendication 1, à savoir l'acide (3S,4S)-3-[2-(2-aminothiazol-4-yl)-(Z)-2-(1-carboxyméthoxyimino)]acétamido-4-succinimido-2-azétidinone-1-sulfonique, ou un sel pharmaceutiquement acceptable de ce composé.

6. Procédé de production d'un composé conforme à la revendication 1, qui comprend la sulfonation d'un dérivé de la 2-azétidinone, de formule:

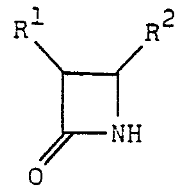

dans laquelle les symboles sont tels que définis dans la revendication 1, ou d'un sel ou d'un dérivé silyle de ce dérivé.

7. Procédé de production d'un composé conforme à la revendication 1, dans lequel $R^1$ est un groupe amino acylé et $R^2$ est tel que défini dans la revendication 1, qui comprend l'acylation d'un dérivé de la 3-amino-2-azétidinone de formule:

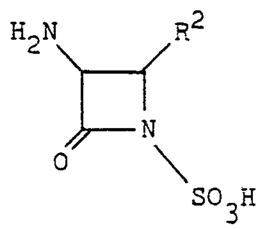

dans laquelle $R^2$ est tel que défini dans la revendication 1, ou d'un sel ou d'un dérivé silyle de ce dérivé.

8. Composition pharmaceutique comprenant un composé conforme à la revendication 1, dans lequel $R^1$ est un groupe amino acylé et $R^2$ est tel que défini dans la revendication 1 en tant qu'ingrédient efficace.